(19) Europäisches Patentamt
European Patent Office
Office européen des brevets

(11) EP 4 168 413 B1

(12) **EUROPEAN PATENT SPECIFICATION**

(45) Date of publication and mention of the grant of the patent:
**12.06.2024 Bulletin 2024/24**

(21) Application number: **21733815.1**

(22) Date of filing: **18.06.2021**

(51) International Patent Classification (IPC):
***C07D 498/22*** *(2006.01)* ***A61K 31/4162*** *(2006.01)*
***A61P 35/00*** *(2006.01)*

(52) Cooperative Patent Classification (CPC):
**C07D 498/22; A61P 35/00**

(86) International application number:
**PCT/EP2021/066655**

(87) International publication number:
**WO 2021/255258 (23.12.2021 Gazette 2021/51)**

(54) **N-LINKED MACROCYCLIC 4-(PYRAZOL-5-YL)-INDOLE DERIVATIVES AS INHIBITORS OF MCL-1**

N-VERKNÜPFTE MAKROCYCLISCHE 4-(PYRAZOL-5-YL)-INDOL-DERIVATE ALS INHIBITOREN VON MCL-1

DÉRIVÉS MACROCYCLIQUES À LIAISON DE 4-(PYRAZOL-5-YL)-INDOLE EN TANT QU'INHIBITEURS DE MCL-1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.06.2020 EP 20181144**

(43) Date of publication of application:
**26.04.2023 Bulletin 2023/17**

(73) Proprietor: **JANSSEN Pharmaceutica NV**
**2340 Beerse (BE)**

(72) Inventors:
- **PESCHIULLI, Aldo**
  **2340 Beerse (BE)**
- **ROMBOUTS, Frederik, Jan, Rita**
  **2340 Beerse (BE)**
- **VELTER, Adriana-Ingrid**
  **2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2017/182625** **WO-A1-2018/178226**
**WO-A1-2020/063792** **WO-A1-2020/103864**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

FIELD OF THE INVENTION

The present invention relates to pharmaceutical agents useful for therapy and/or prophylaxis in a subject, pharmaceutical composition comprising such compounds, and their use as MCL-1 inhibitors, useful for treating or preventing diseases such as cancer.

BACKGROUND OF THE INVENTION

Cellular apoptosis or programmed cell death is critical to the development and homeostasis of many organs including the hematopoietic system. Apoptosis can be initiated via the extrinsic pathway, which is mediated by death receptors, or by the intrinsic pathway using the B cell lymphoma (BCL-2) family of proteins. Myeloid cell leukemia-1 (MCL-1) is a member of the BCL-2 family of cell survival regulators and is a critical mediator of the intrinsic apoptosis pathway. MCL-1 is one of five principal anti-apoptotic BCL-2 proteins (MCL-1, BCL-2, BCL-XL, BCL-w, and BFL1/A1) responsible for maintaining cell survival. MCL-1 continuously and directly represses the activity of the pro-apoptotic BCL-2 family proteins Bak and Bax and indirectly blocks apoptosis by sequestering BH3 only apoptotic sensitizer proteins such as Bim and Noxa. The activation of Bak/Bax following various types of cellular stress leads to aggregation on the mitochondrial outer membrane and this aggregation facilitates pore formation, loss of mitochondrial outer membrane potential, and subsequent release of cytochrome C into the cytosol. Cytosolic cytochrome C binds Apaf-1 and initiates recruitment of procaspase 9 to form apoptosome structures (Cheng et al. eLife 2016; 5: e17755). The assembly of apoptosomes activates the executioner cysteine proteases 3/7 and these effector caspases then cleave a variety of cytoplasmic and nuclear proteins to induce cell death (Julian et al. Cell Death and Differentiation 2017; 24, 1380-1389).

Avoiding apoptosis is an established hallmark of cancer development and facilitates the survival of tumor cells that would otherwise be eliminated due to oncogenic stresses, growth factor deprivation, or DNA damage (Hanahan and Weinberg. Cell 2011;1-44). Thus, unsurprisingly, MCL-1 is highly upregulated in many solid and hematologic cancers relative to normal non-transformed tissue counterparts. The overexpression of MCL-1 has been implicated in the pathogenesis of several cancers where it correlated with poor outcome, relapse, and aggressive disease. Additionally, overexpression of MCL-1 has been implicated in the pathogenesis of the following cancers: prostate, lung, pancreatic, breast, ovarian, cervical, melanoma, B-cell chronic lymphocytic leukemia (CLL), acute myeloid leukemia (AML), and acute lymphoblastic leukemia (ALL). The human MCL-1 genetic locus (1q21) is frequently amplified in tumors and quantitatively increases total MCL-1 protein levels (Beroukhim et al. Nature 2010;463 (7283) 899-905). MCL-1 also mediates resistance to conventional cancer therapeutics and is transcriptionally upregulated in response to inhibition of BCL-2 function (Yecies et al. Blood 2010;115 (16)3304-3313).

A small molecule BH3 inhibitor of BCL-2 has demonstrated clinical efficacy in patients with chronic lymphocytic leukemia and is FDA approved for patients with CLL or AML (Roberts et al. NEJM 2016;374:311-322). The clinical success of BCL-2 antagonism led to the development of several MCL-1 BH3 mimetics that show efficacy in preclinical models of both hematologic malignancies and solid tumors (Kotschy et al. Nature 2016;538 477-486, Merino et al. Sci. Transl. Med;2017 (9)).

MCL-1 regulates several cellular processes in addition to its canonical role in mediating cell survival including mitochondrial integrity and non-homologous end joining following DNA damage (Chen et al. JCI 2018;128(1):500-516). The genetic loss of MCL-1 shows a range of phenotypes depending on the developmental timing and tissue deletion. MCL-1 knockout models reveal there are multiple roles for MCL-1 and loss of function impacts a wide range of phenotypes. Global MCL-1-deficient mice display embryonic lethality and studies using conditional genetic deletion have reported mitochondrial dysfunction, impaired activation of autophagy, reductions in B and T lymphocytes, increased B and T cell apoptosis, and the development of heart failure/ cardiomyopathy (Wang et al. Genes and Dev 2013;27 1351-1364, Steimer et al. Blood 2009;(113) 2805-2815).

WO2018178226 discloses MCL-1 inhibitors and methods of use thereof.

WO2017182625 discloses macrocyclic MCL-1 inhibitors for treating cancer.

WO2018178227 discloses the synthesis of MCL-1 inhibitors.

WO2020063792 discloses indole macrocyclic derivatives.

WO2020103864 discloses macrocyclic indoles as MCL-1 inhibitors.

There remains a need for MCL-1 inhibitors, useful for the treatment or prevention of cancers such as prostate, lung, pancreatic, breast, ovarian, cervical, melanoma, B-cell chronic lymphocytic leukemia (CLL), acute myeloid leukemia (AML), and acute lymphoblastic leukemia (ALL).

SUMMARY OF THE INVENTION

The present invention concerns novel compounds of Formula (I):

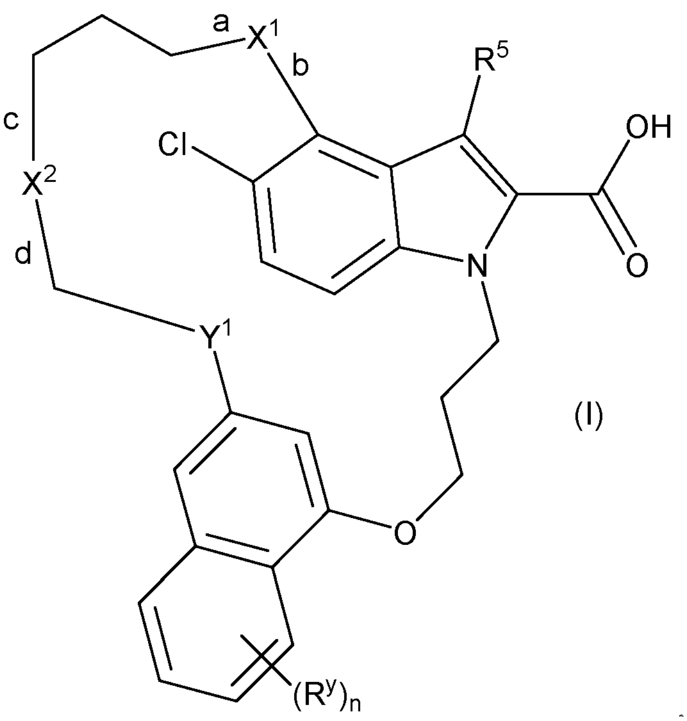

(I)

and the tautomers and the stereoisomeric forms thereof, wherein

$X^1$ represents

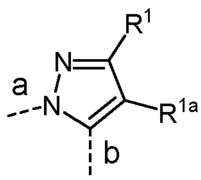

,

wherein 'a' and 'b' indicate how variable $X^1$ is attached to the remainder of the molecule;

$R^y$ represents halo;

n represents 0, 1 or 2;

$X^2$ represents

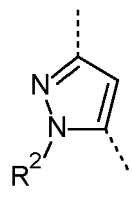

which can be attached to the remainder of the molecule in both directions;

or $X^2$ represents

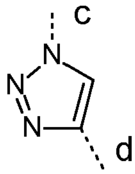
or
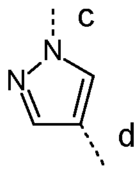

wherein 'c' and ' d' indicate how variable $X^2$ is attached to the remainder of the molecule;

$R^1$ represents hydrogen; or $C_{1-6}$alkyl optionally substituted with one substituent selected from the group consisting of $Het^1$, $-OR^3$, and $-NR^{4a}R^{4b}$;

$R^2$ represents hydrogen; methyl; or $C_{2-6}$alkyl optionally substituted with one substituent selected from the group consisting of $Het^1$, $-OR^3$, and $-NR^{4a}R^{4b}$;

$R^{1a}$ represents methyl or ethyl;

$R^3$ represents hydrogen, $C_{1-4}$alkyl, or $-C_{2-4}$alkyl-O-$C_{1-4}$alkyl;

$R^{4a}$ and $R^{4b}$ are each independently selected from the group consisting of hydrogen and $C_{1-4}$alkyl;

$R^5$ represents hydrogen; methyl; or $C_{2-6}$alkyl optionally substituted with one substituent selected from the group consisting of $C_{3-6}$cycloalkyl, $Het^1$, $-NR^{4a}R^{4b}$, and $-OR^3$;

$Het^1$ represents a 4- to 7-membered monocyclic fully saturated heterocyclyl containing one or two heteroatoms each independently selected from O, S, and N, wherein said S-atom might be substituted to form S(=O) or $S(=O)_2$; wherein said heterocyclyl is optionally substituted with one or two substituents each independently selected from the group consisting of halo, cyano, and -O-$C_{1-4}$alkyl;

$Y^1$ represents $-(CH_2)_m-$ or -S-;

m represents 1 or 2;

and the pharmaceutically acceptable salts and the solvates thereof.

The present invention also relates to a pharmaceutical composition comprising a therapeutically effective amount of a compound of Formula (I), a pharmaceutically acceptable salt, or a solvate thereof, and a pharmaceutically acceptable carrier or excipient.

Additionally, the invention relates to a compound of Formula (I), a pharmaceutically acceptable salt, or a solvate thereof, for use as a medicament, and to a compound of Formula (I), a pharmaceutically acceptable salt, or a solvate thereof, for use in the treatment or in the prevention of cancer.

In a particular embodiment, the invention relates to a compound of Formula (I), a pharmaceutically acceptable salt, or a solvate thereof, for use in the treatment or in the prevention of cancer.

The invention also relates to the use of a compound of Formula (I), a pharmaceutically acceptable salt, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable salt, or a solvate thereof.

The invention also relates to a product comprising a compound of Formula (I), a pharmaceutically acceptable salt, or a solvate thereof, and an additional pharmaceutical agent, as a combined preparation for simultaneous, separate or sequential use in the treatment or prevention of cancer.

DETAILED DESCRIPTION OF THE INVENTION

The term 'halo' or 'halogen' as used herein represents fluoro, chloro, bromo and iodo.

The prefix '$C_{x-y}$' (where x and y are integers) as used herein refers to the number of carbon atoms in a given group. Thus, a $C_{1-6}$alkyl group contains from 1 to 6 carbon atoms, and so on.

The term '$C_{1-4}$alkyl' as used herein as a group or part of a group represents a straight or branched chain fully saturated hydrocarbon radical having from 1 to 4 carbon atoms, such as methyl, ethyl, n-propyl, isopropyl, n-butyl, s-butyl, t-butyl and the like.

The term '$C_{1-6}$alkyl' as used herein as a group or part of a group represents a straight or branched chain fully saturated hydrocarbon radical having from 1 to 6 carbon atoms, such as methyl, ethyl, n-propyl, isopropyl, n-butyl, s-butyl, t-butyl, n-pentyl, *n*-hexyl and the like.

The term '$C_{2-4}$alkyl' as used herein as a group or part of a group represents a straight or branched chain fully saturated hydrocarbon radical having from 2 to 4 carbon atoms, such as ethyl, n-propyl, isopropyl, n-butyl, s-butyl, t-butyl and the like.

The term '$C_{2-6}$alkyl' as used herein as a group or part of a group represents a straight or branched chain fully saturated hydrocarbon radical having from 2 to 6 carbon atoms, such as ethyl, n-propyl, isopropyl, n-butyl, s-butyl, t-butyl, n-pentyl, n-hexyl and the like.

The term '$C_{3-6}$cycloalkyl' as used herein as a group or part of a group defines a fully saturated, cyclic hydrocarbon radical having from 3 to 6 carbon atoms, such as cyclopropyl, cyclobutyl, cyclopentyl and cyclohexyl.

It will be clear for the skilled person that $S(=O)_2$ or $SO_2$ represents a sulfonyl moiety.

It will be clear for the skilled person that CO or C(=O) represents a carbonyl moiety.

Non-limiting examples of 4- to 7-membered monocyclic fully saturated heterocyclyl containing one or two heteroatoms each independently selected from O, S, and N, include, but are not limited to tetrahydropyranyl, tetrahydrofuranyl, morpholinyl, 1,4-dioxanyl, oxetanyl, pyrrolidinyl, piperidinyl, piperazinyl, and azetidinyl.

In general, whenever the term 'substituted' is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture.

Combinations of substituents and/or variables are permissible only if such combinations result in chemically stable compounds. 'Stable compound' is meant to indicate a compound that is sufficiently robust to survive isolation to a useful degree of purity from a reaction mixture.

The skilled person will understand that the term 'optionally substituted' means that the atom or radical indicated in the expression using 'optionally substituted' may or may not be substituted (this means substituted or unsubstituted respectively).

When two or more substituents are present on a moiety they may, where possible and unless otherwise indicated or clear from the context, replace hydrogens on the same atom or they may replace hydrogen atoms on different atoms in the moiety.

$Het^1$ may be attached to the remainder of the molecule of Formula (I) through any available ring carbon or nitrogen atom as appropriate, if not otherwise specified.

It will be clear that an alternative presentation (with the structure of $X^1$ in the Formula) of a Compound of Formula (I) is

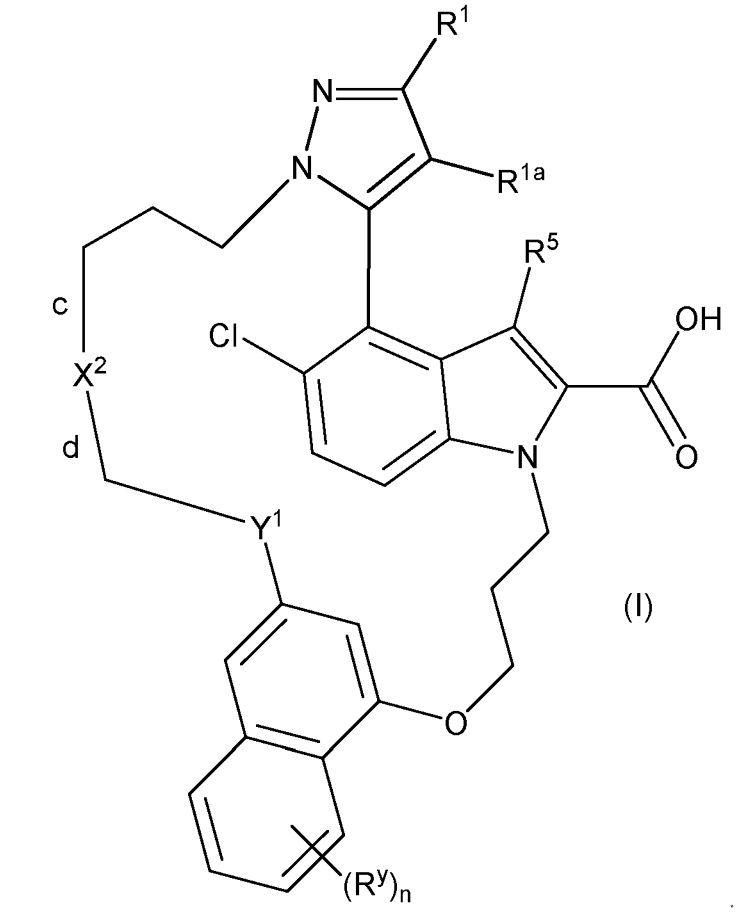

(I)

It will be clear that a Compound of Formula (I) includes Compounds of Formula (I-x) and (I-y) (both directions of $X^2$ being

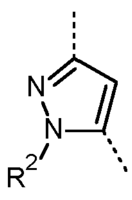

)

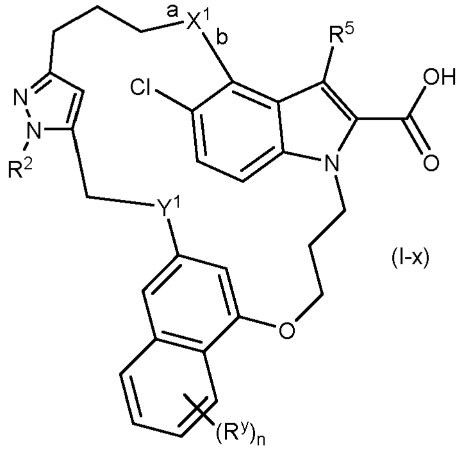

(I-x)

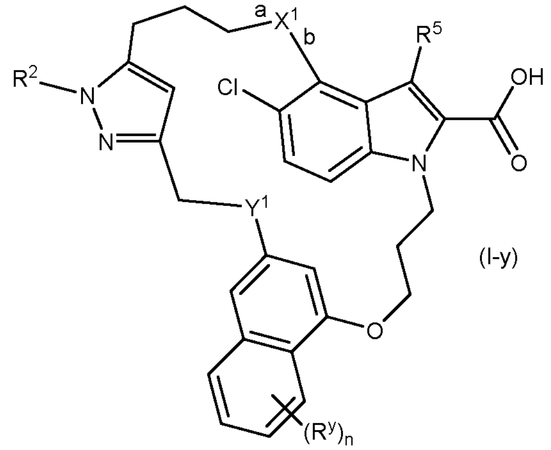

(I-y)

It will be clear that a Compound of Formula (I) includes Compounds of Formula (I-xx) ($X^2$ being

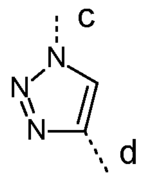

)

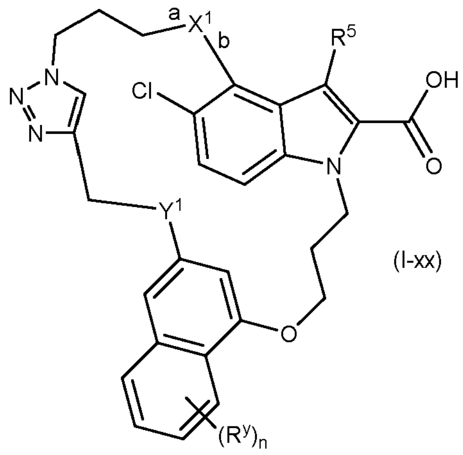

(I-xx)

.

It will be clear that a Compound of Formula (I) includes Compounds of Formula (I-xxx) ($X^2$ being

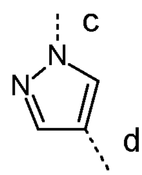

)

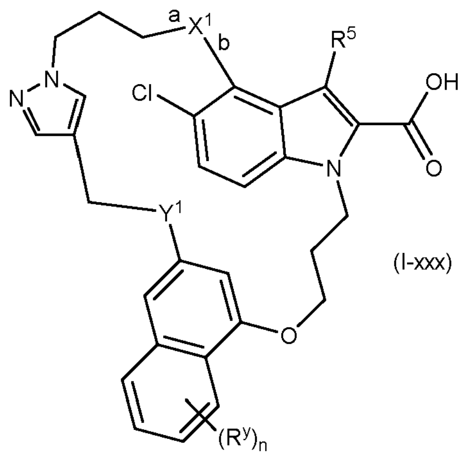

(I-xxx)

.

When any variable occurs more than one time in any constituent, each definition is independent.

When any variable occurs more than one time in any formula (e.g. Formula (I)), each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, or subject (e.g., human) that is

being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R*, *S*) around one or more atoms, contemplates each possible stereoisomer, or mixture of two or more stereoisomers.

Hereinbefore and hereinafter, the term "compound(s) of Formula (I)" is meant to include the tautomers thereof and the stereoisomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present invention.

In particular, the compounds disclosed herein possess axial chirality, by virtue of restricted rotation around a biaryl bond and as such may exist as mixtures of atropisomers. When a compound is a pure atropisomer, the stereochemistry at each chiral center may be specified by either $R_a$ or $S_a$. Such designations may also be used for mixtures that are enriched in one atropisomer. Further description of atropisomerism and axial chirality and rules for assignment of configuration can be found in Eliel, E.L. & Wilen, S. H. 'Stereochemistry of Organic Compounds' John Wiley and Sons, Inc. 1994.

Diastereomers (or diastereoisomers) are stereoisomers that are not enantiomers, i.e. they are not related as mirror images. If a compound contains a double bond, the substituents may be in the *E* or the *Z* configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, atropisomers, diastereomers, racemates, *E* isomers, *Z* isomers, cis isomers, trans isomers and mixtures thereof, whenever chemically possible.

The meaning of all those terms, i.e. enantiomers, atropisomers, diastereomers, racemates, *E* isomers, *Z* isomers, cis isomers, trans isomers and mixtures thereof are known to the skilled person.

The absolute configuration is specified according to the Cahn-Ingold-Prelog system. The configuration at an asymmetric atom is specified by either *R* or *S*. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light. Optically active ($R_a$)- and ($S_a$)-atropisomers may be prepared using chiral synthons, chiral reagents or chiral catalysts, or resolved using conventional techniques well known in the art, such as chiral HPLC.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (*R*), this means that the compound is substantially free of the (*S*) isomer; when a compound of Formula (I) is for instance specified as *E*, this means that the compound is substantially free of the Z isomer; when a compound of Formula (I) is for instance specified as cis, this means that the compound is substantially free of the trans isomer; when a compound of Formula (I) is for instance specified as $R_a$, this means that the compound is substantially free of the $S_a$ atropisomer.

Pharmaceutically acceptable salts, in particular pharmaceutically acceptable additions salts, include acid addition salts and base addition salts. Such salts may be formed by conventional means, for example by reaction of a free acid or a free base form with one or more equivalents of an appropriate base or acid, optionally in a solvent, or in a

medium in which the salt is insoluble, followed by removal of said solvent, or said medium, using standard techniques (e.g. *in vacuo*, by freeze-drying or by filtration). Salts may also be prepared by exchanging a counter-ion of a compound of the invention in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I), and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically.

Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present invention disclosure also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$ , $^{13}N$, $^{15}O$, $^{17}O$, $^{18}O$, $^{32}P$, $^{33}P$, $^{35}S$, $^{18}F$, $^{36}Cl$, $^{122}I$, $^{123}I$, $^{125}I$, $^{131}I$, $^{75}Br$, $^{76}Br$, $^{77}Br$ and $^{82}Br$. Preferably, the isotope is selected from the group of $^{2}H$, $^{3}H$, $^{11}C$ and $^{18}F$. More preferably, the isotope is $^{2}H$. In particular, deuterated compounds are intended to be included within the scope of the present invention.

Certain isotopically-labeled compounds of the present invention (e.g., those labeled with $^{3}H$ and $^{14}C$) may be useful for example in substrate tissue distribution assays. Tritiated ($^{3}H$) and carbon-14 ($^{14}C$) isotopes are useful for their ease of preparation and detectability. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

The present invention relates in particular to compounds of Formula (I) as defined herein, and the tautomers and the stereoisomeric forms thereof, wherein $X^1$ represents

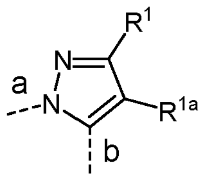

,

wherein 'a' and 'b' indicate how variable $X^1$ is attached to the remainder of the molecule;

$R^y$ represents halo;

n represents 0 or 1;

$X^2$ represents

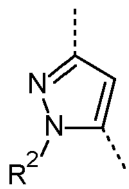

which can be attached to the remainder of the molecule in both directions;

or $X^2$ represents

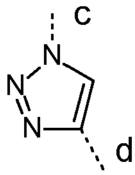

or

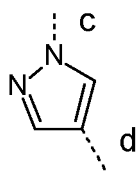

,

wherein 'c' and ' d' indicate how variable $X^2$ is attached to the remainder of the molecule;

$R^1$ represents methyl;

$R^2$ represents methyl;

$R^{1a}$ represents methyl;

$R^5$ represents methyl;

$Y^1$ represents $-(CH_2)_m-$ or -S-;

m represents 1;

and the pharmaceutically acceptable salts and the solvates thereof.

The present invention relates in particular to compounds of Formula (I) as defined herein, and the tautomers

and the stereoisomeric forms thereof, wherein

$X^1$ represents

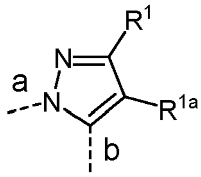

,

wherein 'a' and 'b' indicate how variable $X^1$ is attached to the remainder of the molecule;

$R^y$ represents halo;

n represents 0, 1 or 2;

$X^2$ represents

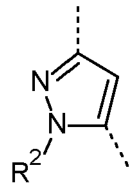

which can be attached to the remainder of the molecule in both directions;

$R^1$ represents hydrogen; or $C_{1-6}$alkyl optionally substituted with one substituent selected from the group consisting of $Het^1$, $-OR^3$, and $-NR^{4a}R^{4b}$;

$R^2$ represents hydrogen; methyl; or $C_{2-6}$alkyl optionally substituted with one substituent selected from the group consisting of $Het^1$, $-OR^3$, and $-NR^{4a}R^{4b}$;

$R^{1a}$ represents methyl or ethyl;

$R^3$ represents hydrogen, $C_{1-4}$alkyl, or $-C_{2-4}$alkyl-O-$C_{1-4}$alkyl;

$R^{4a}$ and $R^{4b}$ are each independently selected from the group consisting of hydrogen and $C_{1-4}$alkyl;

$R^5$ represents hydrogen; methyl; or $C_{2-6}$alkyl optionally substituted with one substituent selected from the group consisting of $C_{3-6}$cycloalkyl, $Het^1$, $-NR^{4a}R^{4b}$, and $-OR^3$;

$Het^1$ represents a 4- to 7-membered monocyclic fully saturated heterocyclyl containing one or two heteroatoms each independently selected from O, S, and N, wherein said S-atom might be substituted to form S(=O) or $S(=O)_2$; wherein said heterocyclyl is optionally substituted with one or two substituents each independently selected from the group consisting of halo, cyano, and -O-$C_{1-4}$alkyl;

$Y^1$ represents $-(CH_2)_m-$ or -S-;

m represents 1 or 2;

and the pharmaceutically acceptable salts and the solvates thereof.

The present invention relates in particular to compounds of Formula (I) as defined herein, and the tautomers and the stereoisomeric forms thereof, wherein

$X^1$ represents

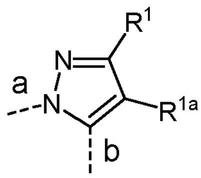

,

wherein 'a' and 'b' indicate how variable $X^1$ is attached to the remainder of the molecule;

$R^y$ represents halo;

n represents 0 or 1;

$X^2$ represents

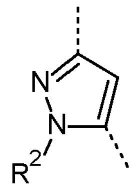

which can be attached to the remainder of the molecule in both directions;

$R^1$ represents methyl;

$R^2$ represents methyl;

$R^{1a}$ represents methyl;

$R^5$ represents methyl;

$Y^1$ represents $-(CH_2)_m-$;

m represents 1;

and the pharmaceutically acceptable salts and the solvates thereof.

The present invention relates in particular to compounds of Formula (I) as defined herein, and the tautomers and the stereoisomeric forms thereof, wherein

$X^1$ represents

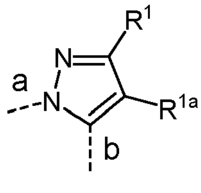

,

wherein 'a' and 'b' indicate how variable $X^1$ is attached to the remainder of the molecule;
$R^y$ represents fluoro;
n represents 1;

$X^2$ represents

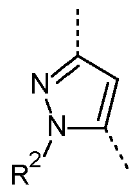

which can be attached to the remainder of the molecule in both directions;
or $X^2$ represents

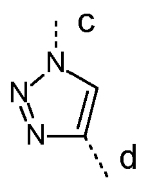

or

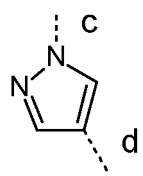

,

wherein 'c' and ' d' indicate how variable $X^2$ is attached to the remainder of the molecule;
$R^1$ represents methyl;
$R^2$ represents methyl;
$R^{1a}$ represents methyl;
$R^5$ represents methyl;
$Y^1$ represents $-(CH_2)_m-$ or -S-;
m represents 1;
and the pharmaceutically acceptable salts and the solvates thereof.

The present invention relates in particular to compounds of Formula (I) as defined herein, and the tautomers and the stereoisomeric forms thereof, wherein

$X^1$ represents

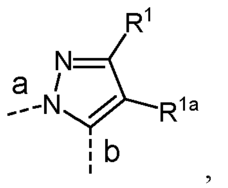

,

wherein 'a' and 'b' indicate how variable $X^1$ is attached to the remainder of the molecule;
$R^y$ represents fluoro;
n represents 1;
$X^2$ represents

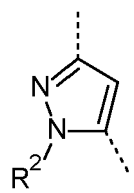

which can be attached to the remainder of the molecule in both directions;
$R^1$ represents methyl;

$R^2$ represents methyl;
$R^{1a}$ represents methyl;
$R^5$ represents methyl;
$Y^1$ represents $-(CH_2)_m-$;
m represents 1;
and the pharmaceutically acceptable salts and the solvates thereof.

In an embodiment, the present invention relates to those compounds of Formula (I) and the pharmaceutically acceptable salts, and the solvates thereof, or any subgroup thereof as mentioned in any of the other embodiments, wherein $Y^1$ represents -S-.

In an embodiment, the present invention relates to those compounds of Formula (I) and the pharmaceutically acceptable salts, and the solvates thereof, or any subgroup thereof as mentioned in any of the other embodiments, wherein $R^y$ represents fluoro.

In an embodiment, the present invention relates to those compounds of Formula (I) and the pharmaceutically acceptable salts, and the solvates thereof, or any subgroup thereof as mentioned in any of the other embodiments, wherein

n represents 1; and
$R^y$ represents fluoro.

In an embodiment, the present invention relates to those compounds of Formula (I) and the pharmaceutically acceptable salts, and the solvates thereof, or any subgroup thereof as mentioned in any of the other embodiments, wherein $R^1$ represents hydrogen.

In an embodiment, the present invention relates to those compounds of Formula (I) and the pharmaceutically acceptable salts, and the solvates thereof, or any subgroup thereof as mentioned in any of the other embodiments, wherein $R^1$ represents methyl.

In an embodiment, the present invention relates to those compounds of Formula (I) and the pharmaceutically acceptable salts, and the solvates thereof, or any subgroup thereof as mentioned in any of the other embodiments, wherein $R^2$ represents methyl.

In an embodiment, the present invention relates to those compounds of Formula (I) and the pharmaceutically acceptable salts, and the solvates thereof, or any subgroup thereof as mentioned in any of the other embodiments, wherein $R^5$ represents methyl.

In an embodiment, the present invention relates to those compounds of Formula (I) and the pharmaceutically acceptable salts, and the solvates thereof, or any subgroup thereof as mentioned in any of the other embodiments, wherein $R^5$ represents ethyl.

In an embodiment, the present invention relates to those compounds of Formula (I) and the pharmaceutically acceptable salts, and the solvates thereof, or any subgroup thereof as mentioned in any of the other embodiments, wherein $R^5$ represents methyl; or $C_{2-6}$alkyl optionally substituted with $C_{3-6}$cycloalkyl or $Het^1$.

In an embodiment, the present invention relates to those compounds of Formula (I) and the pharmaceutically acceptable salts, and the solvates thereof, or any subgroup thereof as mentioned in any of the other embodiments, wherein $R^5$ represents methyl; or $C_{2-6}$alkyl optionally substituted with one $-OR^3$.

In an embodiment, the present invention relates to those compounds of Formula (I) and the pharmaceutically acceptable salts, and the solvates thereof, or any subgroup thereof as mentioned in any of the other embodiments, wherein $R^5$ represents $C_{2-6}$alkyl optionally substituted with one $-OR^3$.

In an embodiment, the present invention relates to those compounds of Formula (I) and the pharmaceutically acceptable salts, and the solvates thereof, or any subgroup thereof as mentioned in any of the other embodiments, wherein

$R^5$ represents $C_{2-6}$alkyl optionally substituted with one $-OR^3$; and
$R^3$ represents $-C_{2-4}$alkyl-O-$C_{1-4}$alkyl.

In an embodiment, the present invention relates to those compounds of Formula (I) and the pharmaceutically acceptable salts, and the solvates thereof, or any subgroup thereof as mentioned in any of the other embodiments, wherein

$R^5$ represents $C_{2-6}$alkyl substituted with one $-OR^3$; and
$R^3$ represents $-C_{2-4}$alkyl-O-$C_{1-4}$alkyl.

In an embodiment, the present invention relates to those compounds of Formula (I) and the pharmaceutically acceptable salts, and the solvates thereof, or any subgroup thereof as mentioned in any of the other embodiments, wherein n represents 0.

In an embodiment, the present invention relates to those compounds of Formula (I) and the pharmaceutically acceptable salts, and the solvates thereof, or any subgroup thereof as mentioned in any of the other embodiments, wherein n represents 1.

In an embodiment, the present invention relates to those compounds of Formula (I) and the pharmaceutically acceptable salts, and the solvates thereof, or any subgroup thereof as mentioned in any of the other embodiments, wherein n represents 2.

In an embodiment, the present invention relates to those compounds of Formula (I) and the pharmaceutically acceptable salts, and the solvates thereof, or any subgroup thereof as mentioned in any of the other embodiments, wherein m represents 1.

In an embodiment, the present invention relates to those compounds of Formula (I) and the pharmaceutically acceptable salts, and the solvates thereof, or any subgroup thereof as mentioned in any of the other embodiments, wherein m represents 2.

In an embodiment, the present invention relates to those compounds of Formula (I) and the pharmaceutically acceptable salts, and the solvates thereof, or any subgroup thereof as mentioned in any of the other embodiments, wherein $Het^1$ is attached to the remainder of the molecule of Formula (I) through a nitrogen atom.

In an embodiment, the present invention relates to those compounds of Formula (I) and the pharmaceutically acceptable salts, and the solvates thereof, or any subgroup thereof as mentioned in any of the other embodiments, wherein n is 1 and wherein $R^y$ is in position 3 as indicated below:

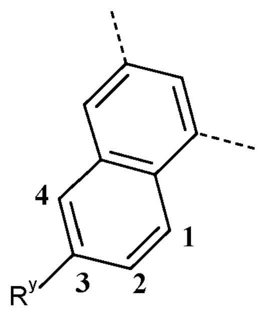

In an embodiment, the present invention relates to those compounds of Formula (I) and the pharmaceutically acceptable salts, and the solvates thereof, or any subgroup thereof as mentioned in any of the other embodiments, wherein n is 1 and wherein $R^y$ is in position 3 as indicated below; and wherein $R^y$ represents fluoro:

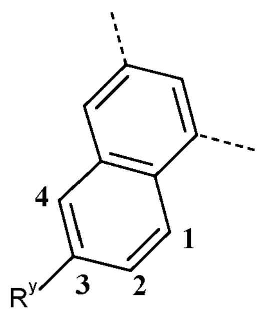

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-x):

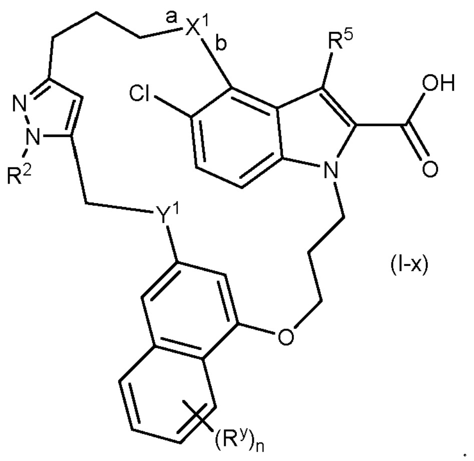

(I-x)

It will be clear that all variables in the structure of Formula (I-x), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-y):

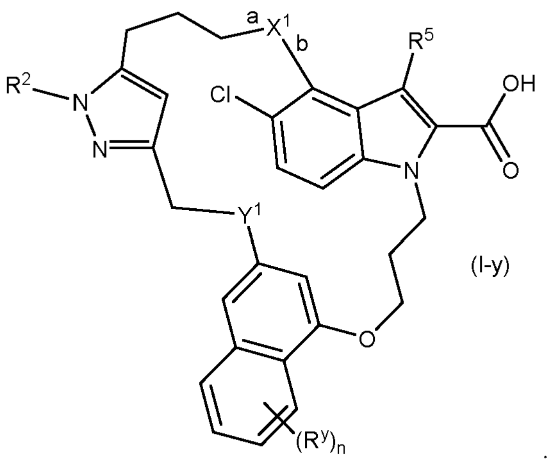

(I-y)

It will be clear that all variables in the structure of Formula (I-y), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-xx):

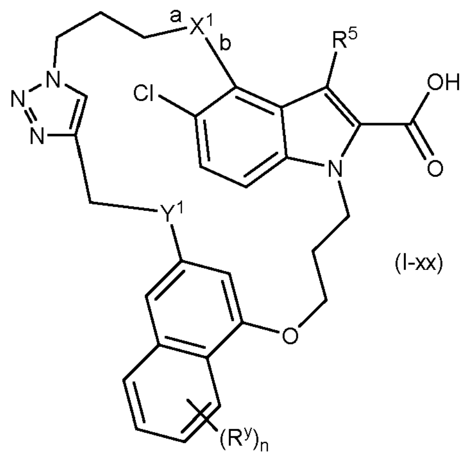

(I-xx)

It will be clear that all variables in the structure of Formula (I-xx), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-xxx):

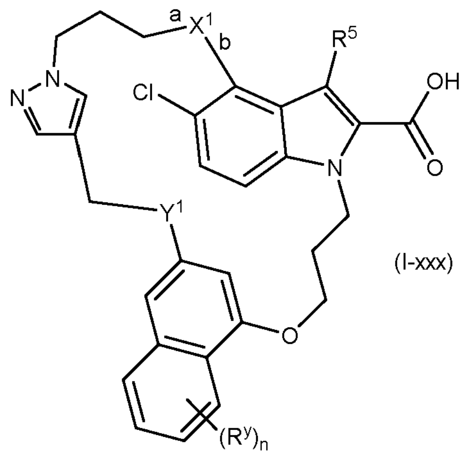

(I-xxx)

It will be clear that all variables in the structure of Formula (I-xxx), are defined as defined for the compounds of Formula (I) or any subgroup thereof as mentioned in any of the other embodiments.

In an embodiment, the present invention relates to a subgroup of Formula (I) as defined in the general reaction schemes.

In an embodiment the compound of Formula (I) is selected from the group consisting of any of the exemplified compounds,

tautomers and stereoisomeric forms thereof,
any pharmaceutically acceptable salts, and the solvates thereof.

All possible combinations of the above indicated embodiments are considered to be embraced within the scope of the invention.

METHODS FOR THE PREPARATION OF COMPOUNDS

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or prepared by standard synthetic processes commonly used by those skilled in the art of organic chemistry. The following

schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under $N_2$-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.

Compounds of Formula (I) can be prepared according to Scheme 1,

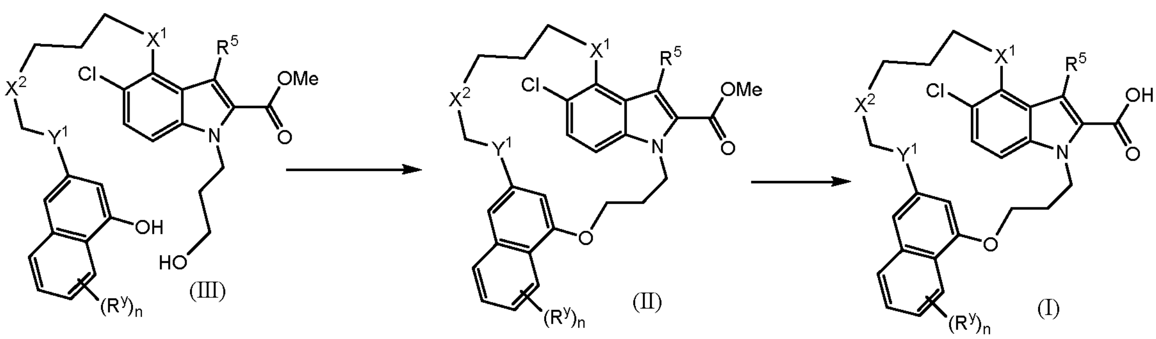

Scheme 1

- by reacting an intermediate of Formula (II) where $X^1$, $X^2$, $Y^1$, $R^5$, and $(R^y)_n$ are defined as in Formula (I), with a suitable base such as, for example, LiOH or NaOH, in a suitable solvent such as water or a mixture of water and a suitable organic solvent such as dioxane or THF, or a mixture of MeOH and THF, at a suitable temperature such as room temperature or 60 °C.
- Intermediates of Formula (II) can be prepared by reacting an intermediate of Formula (III) with a suitable reagent, such as, for example, diethyl azodicarboxylate (DEAD) or di-tert-butyl azodicarboxylate (DTBAD), in the presence of a suitable phosphine such as, for example, $PPh_3$, in a suitable solvent such as, for example, THF, toluene, or a mixture thereof, at a suitable temperature such as, for example, room temperature or 70 °C.

Intermediates of Formula (II-b) and (II-c) wherein $X^1$, $Y^1$, $R^5$, and $(R^y)_n$ are defined as in Formula (I), can be prepared according to Scheme 2,

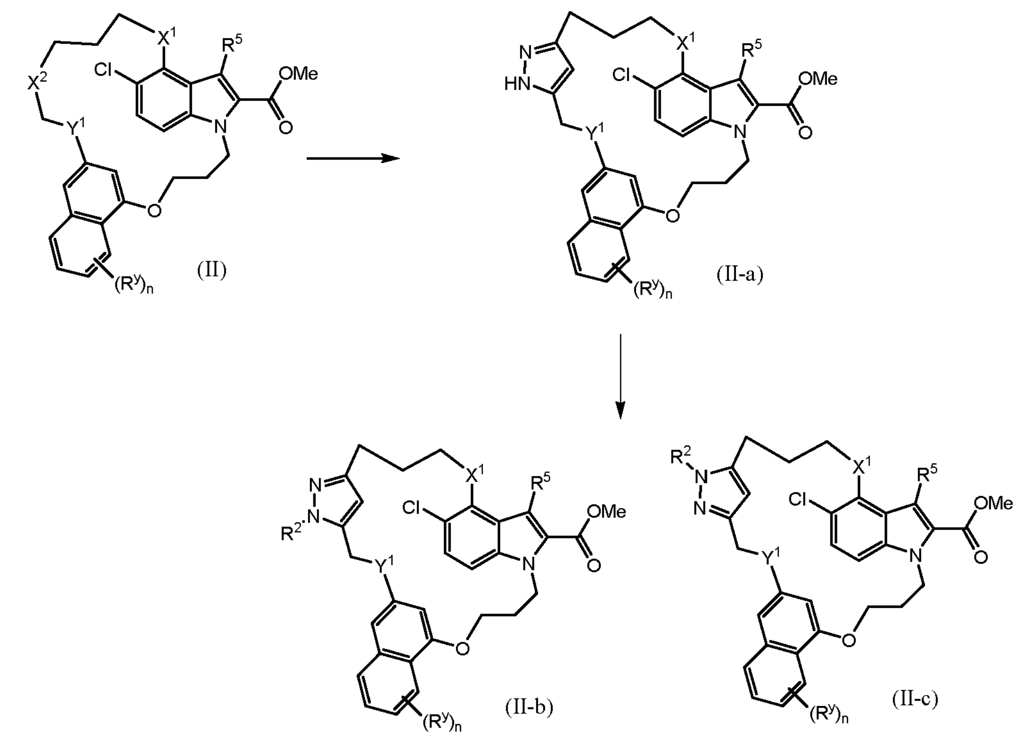

(II) (II-a) (II-b) (II-c)

Scheme 2.

- by reacting an intermediate of Formula (II-a) with a suitable alkylating agent $R^2L$ such as, for example, an alkyl halide, in a suitable solvent such as, for example, DMF, or acetonitrile, in the presence of a suitable base such as, for example, trietylamine ($Et_3N$), N,N-Diisopropylethylamine ($iPr_2EtN$), $Cs_2CO_3$, or 1,8-Diazabicyclo[5.4.0]undec-7-ene (DBU), at a suitable temperature such as, for example, room temperature or 60 °C, followed by a suitable separation of the isomers (II-b) and (II-c) such as, for example, a chromatographic separation.
- Intermediates of Formula (II-a) can be obtained by reacting an intermediate of Formula (II), where $R^2$ is defined as a suitable protecting group such as, for example, tetrahydropyran (THP), with a suitable deprotection reagent such as, for example, HCl, in a suitable solvent such as, for example, dioxane or isopropanol, at a suitable temperature such as, for example, room temperature.

When $X^2$ represents

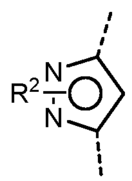

,

intermediates of Formula (III) wherein $X^1$, $Y^1$, $R^5$, and $(R^y)_n$ are defined as in Formula (I), can be prepared according to Scheme 3,

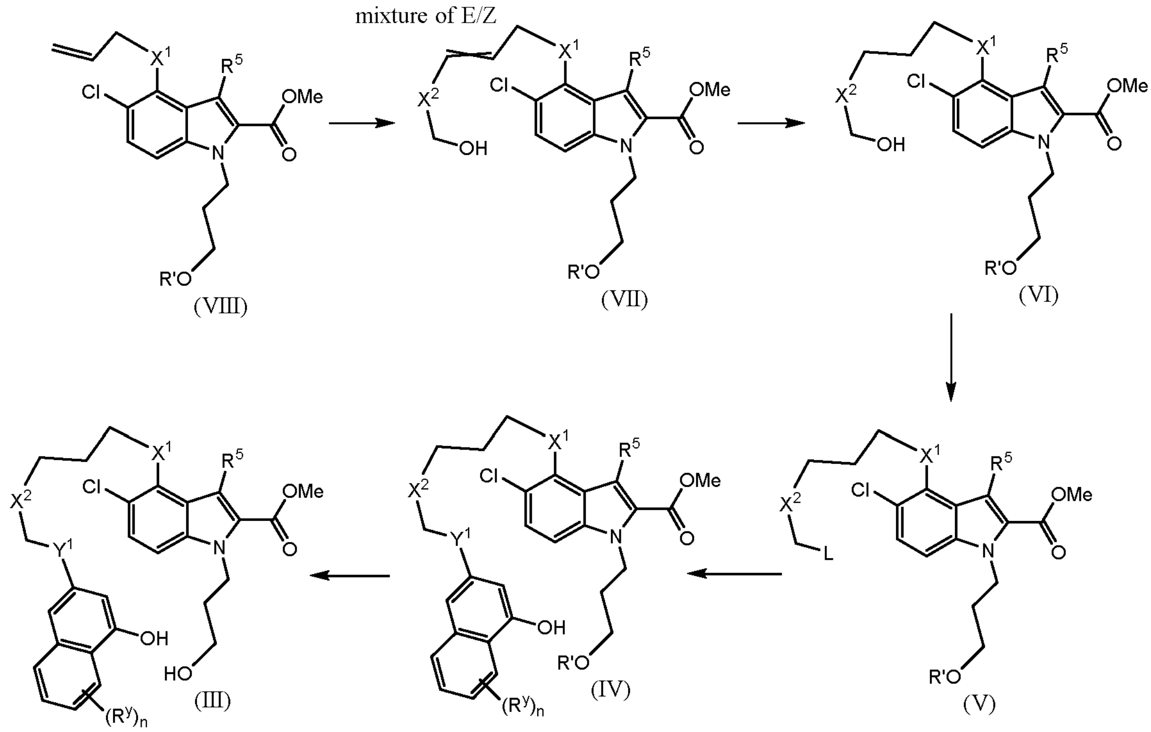


Scheme 3

- by reacting an intermediate of Formula (IV), wherein R' is a suitable protecting group such as tert-butyldimethylsilyl (TBDMS), with a suitable deprotecting agent such as, for example, TBAF in a suitable solvent such as, for example, THF, at a suitable temperature such as, for example, room temperature.
- Intermediates of Formula (IV) can be prepared by reacting an intermediate of Formula (V), where L is a suitable leaving group such as, for example, mesylate (MsO) or Cl, with a suitably substituted 3-(acetylthio)naphthalen-1-yl acetate , in the presence of a suitable base, such as, for example, $K_2CO_3$, in a suitable solvent, such as, for example, methanol or THF, or a mixture thereof, at a suitable temperature, such as, for example, room temperature.
- Intermediates of Formula (V) can be prepared by reacting an intermediate of Formula (VI), with a suitable activating agent such as, for example, mesyl chloride (MsCl) or $SOCl_2$, in the presence of a suitable base such as, for example, trietylamine ($Et_3N$) or di-isopropylethylamine (DIPEA), in a suitable solvent such as DCM, at a suitable temperature such as, for example, room temperature.
- Intermediates of Formula (VI) can be prepared by reacting an intermediate of Formula (VII) with a suitable hydrogenating agent such as, for example, hydrogen, in the presence of a suitable catalyst such as, for example, Pd/C, in a suitable solvent such as, for example, ethyl acetate (EtOAc), at a suitable temperature such as, for example, room temperature.
- Intermediates of Formula (VII) can be prepared by reacting an intermediate of Formula (VIII) with a suitable pyrazole bromide such as, for example, (3-bromo-1-methyl-1H-pyrazol-5-yl)methanol (CAS [1784533-05-6]), in the presence of a suitable catalyst such as, for example, bis(tri-tert-butylphosphine)palladium(0) ($Pd(tBu_3P)_2$) (CAS [53199-31-8]), in the presence of a suitable base such as, for example, DIPEA, in a suitable solvent such as, for example, *N,N*-dimethylformamide (DMF), at a suitable temperature such as, for example, 120 °C.

It will be clear for a skilled person that an intermediate of Formula (VIII) can also be reacted with a suitable pyrazole bromide carrying an $R^2$ substituent allowing to access compounds of formula II-b and II-c.

Intermediates of Formula (VIII) wherein $X^1$ and $R^5$ are defined as in Formula (I), and R' is a suitable protecting group such as TBDMS, can be prepared according to Scheme 4,

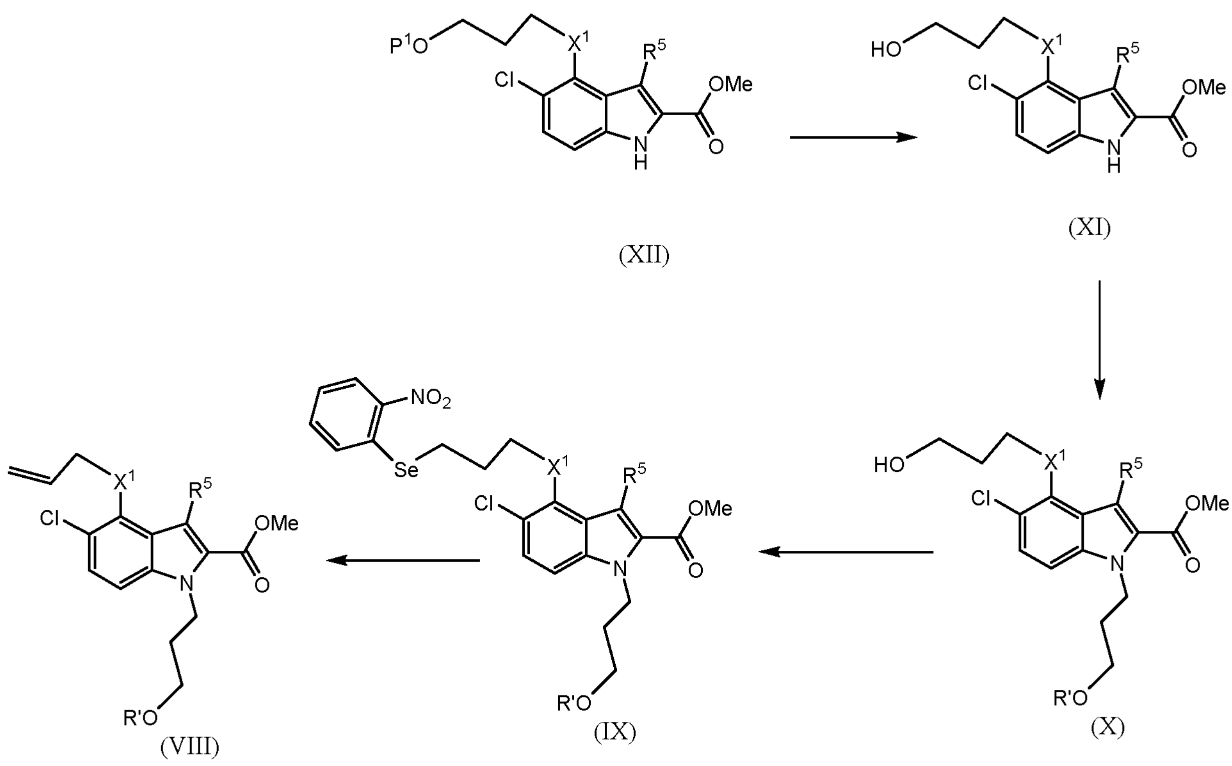

Scheme 4

- by reacting an intermediate of Formula (IX) with a suitable oxidizing agent such as, for example, hydrogen peroxide, in a suitable solvent such as, for example, tetrahydrofuran (THF), at a suitable temperature such as, for example, room temperature.
- Intermediates of Formula (IX) can be prepared by reacting an intermediate of Formula (X) with a suitable dehydration reagent such as, for example, 2-nitrophenyl selenocyanate, in the presence of a suitable activating agent such as, for example, tri-n-butylphosphine, in a suitable solvent such as, for example, THF, at a suitable temperature such as, for example, 0 °C or room temperature.
- Intermediates of Formula (X) can be prepared by reacting an intermediate of Formula (XI) with a suitable O-protected propyl halide or alkylsulfonate such as, for example, (3-bromopropoxy)(tert-butyl)dimethylsilane, in the presence of a suitable base such as, for example, $K_2CO_3$, in a suitable solvent such as, for example, acetonitrile, at a suitable temperature such as, for example, 80 °C.
- Intermediates of Formula (XI) can be prepared by reacting an intermediate of Formula (XII), wherein $P^1$ is a suitable protecting groups such as, for example, TBDMS, with a suitable deprotecting agent such as, for example, HCl, in a suitable solvent such as, for example, 1,4-dioxane, at a suitable temperature such as, for example, room temperature.

Intermediates of Formula (XII) wherein $X^1$ and $R^5$ are defined as in Formula (I), can be prepared according to Scheme 5,

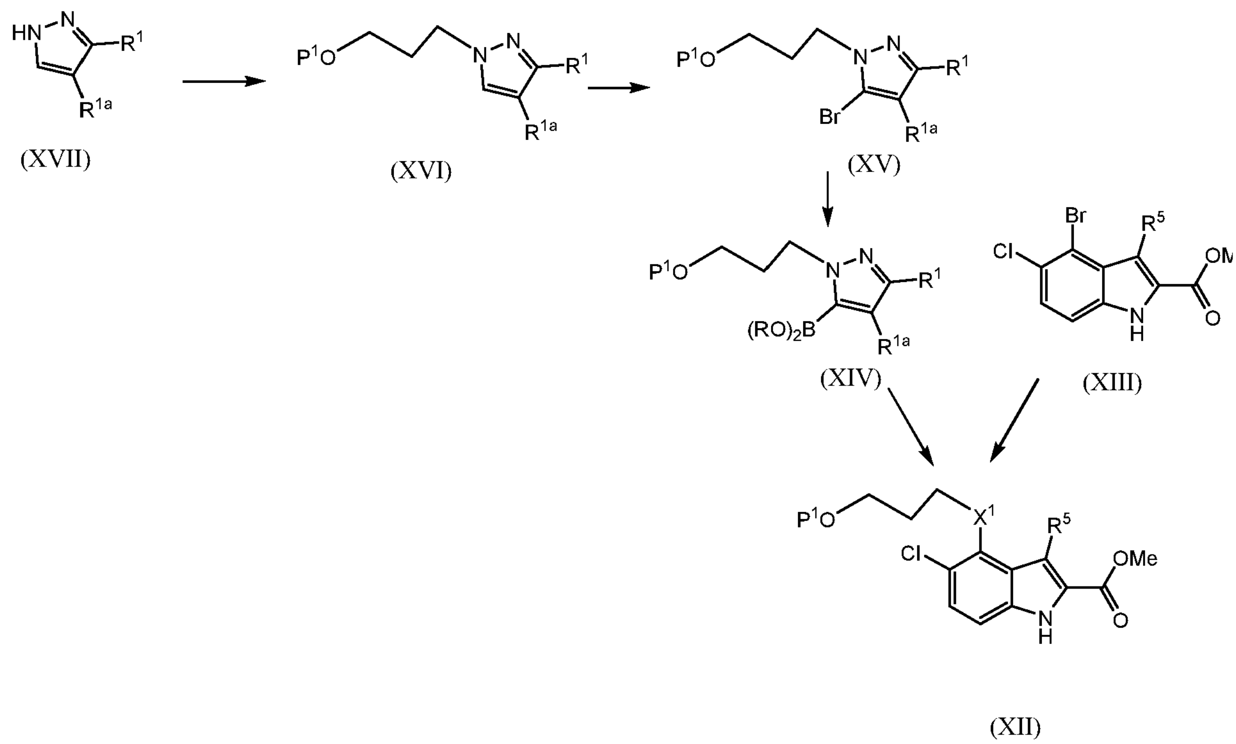

Scheme 5

- by reacting an intermediate of Formula (XIII) with an intermediate of Formula (XIV), wherein $R^1$ and $R^{1a}$ are defined as in Formula (I), in the presence of a suitable base such as, for example, $K_2CO_3$, and a suitable catalyst such as, for example, Pd(amphos)$Cl_2$ (CAS [887919-35-9]), in a suitable solvent such as, for example, a mixture of 1,4-dioxane and water, at a suitable temperature such as, for example, 80 °C.
- Intermediates of Formula (XIV) can be prepared by reacting an intermediate of Formula (XV) with a suitable boronate precursor such as, for example, 2-isopropoxy-4,4,5,5-tetramethyl-1,3,2-dioxaborolane (CAS [61676-62-8]), in the presence of a suitable base such as, for example, BuLi, in a suitable solvent such as, for example, THF, at a suitable temperature such as, for example, -78 °C or room temperature.
- Intermediates of Formula (XV) can be prepared by reacting an intermediate of Formula (XVI) with a suitable brominating agent such as, for example, N-bromosuccinimide, in a suitable solvent such as, for example, DCM, at a suitable temperature such as, for example, 0 °C or room temperature.
- Intermediates of Formula (XVI) can be prepared by reacting a suitable pyrazole (XVII) such as, for example, 3,4-dimethyl-1H-pyrazole (CAS [2820-37-3]), with a suitable protected alkylating agent such as, for example, (3-bromopropoxy)-tert-butyldimethylsilane (CAS [89031-84-5]), in the presence of a suitable base such as, for example, NaH, in a suitable solvent such as, for example, DMF, at a suitable temperature such as, for example, 0 °C or room temperature.
- It will be clear for a skilled person that a mixture of isomers can be obtained during the synthesis of an intermediate of Formula (XVI), of which the desired isomer can be separated using a suitable method such as, for instance, column chromatography.

Intermediates of Formula (XIII) wherein $R^5$ is defined as in Formula (I), can be prepared according to Scheme 6,

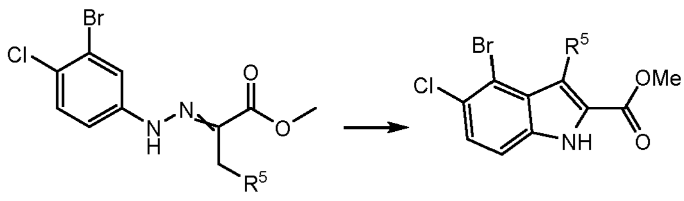

mixture of (E) and (Z) isomers (XVIII) (XIII)

Scheme 6

- by reacting an intermediate of Formula (XVIII), with a suitable acid, such as, for example, sulfuric acid, in a suitable solvent, such as, for example, acetic acid, at a suitable temperature, such as, for example, 70 °C.
- Intermediates of Formula (XVIII) can be prepared by reacting (3-bromo-4-chlorophenyl)hydrazine with a suitable methyl 2-oxoalkanoate such as, for example, methyl 2-oxobutanoate, in the presence of a suitable acid, such as, for example, hydrochloric acid, in a suitable solvent, such as, for example, methanol, at a suitable temperature, such as, for example, 65 °C.

Alternatively, when $X^2$ represents

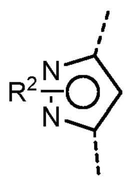

,

an intermediate of Formula (III) wherein $X^1$, $R^5$, and $(R^y)_n$ are defined as in Formula (I), Hal is defined as a suitable haologen, and wherein $Y^1$ is $(CH_2)_m$ and m is defined as in Formula (I), can be prepared according to Scheme 7,

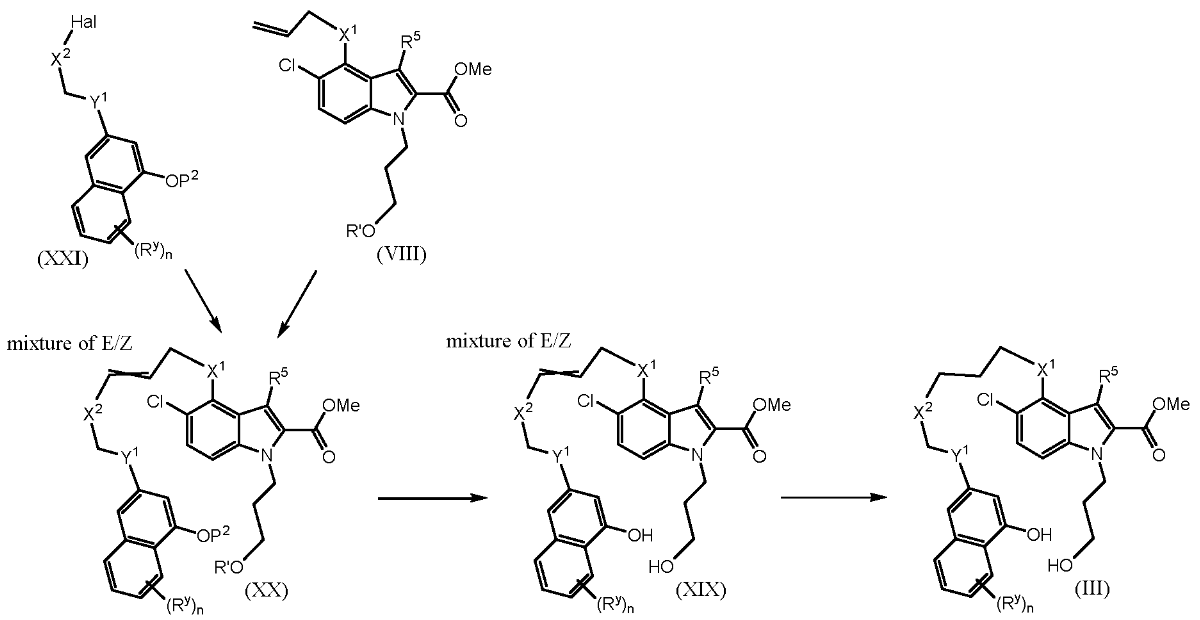


Scheme 7

- by reacting an intermediate of Formula (XIX) with a suitable hydrogenating agent such as, for example, hydrogen, in the presence of a suitable catalyst such as, for example, Pd/C, in a suitable solvent such as, for example, EtOAc, at a suitable temperature such as, for example, room temperature.

- Intermediates of Formula (XIX) can be prepared by reacting an intermediate of Formula (XX) with a suitable deprotecting agent such as, for example, tetrabutylammonium fluoride (TBAF), in a suitable solvent such as, for example, THF, at a suitable temperature such as, for example, room temperature.
- Intermediates of Formula (XX) can be prepared by reacting an intermediate of Formula (VIII) with an intermediate of Formula (XXI), in the presence of a suitable base such as, for example, DIPEA, and a suitable catalyst such as, for example, $Pd(tBu_3P)_2$, in a suitable solvent such as, for example, DMF, at a suitable temperature such as, for example, 120 °C.

Alternatively, when $X^2$ represents

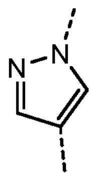

,

intermediates of Formula (III) wherein $X^1$, $R^5$, and $(R^y)_n$ are defined as in Formula (I), can be prepared according to Scheme 8,

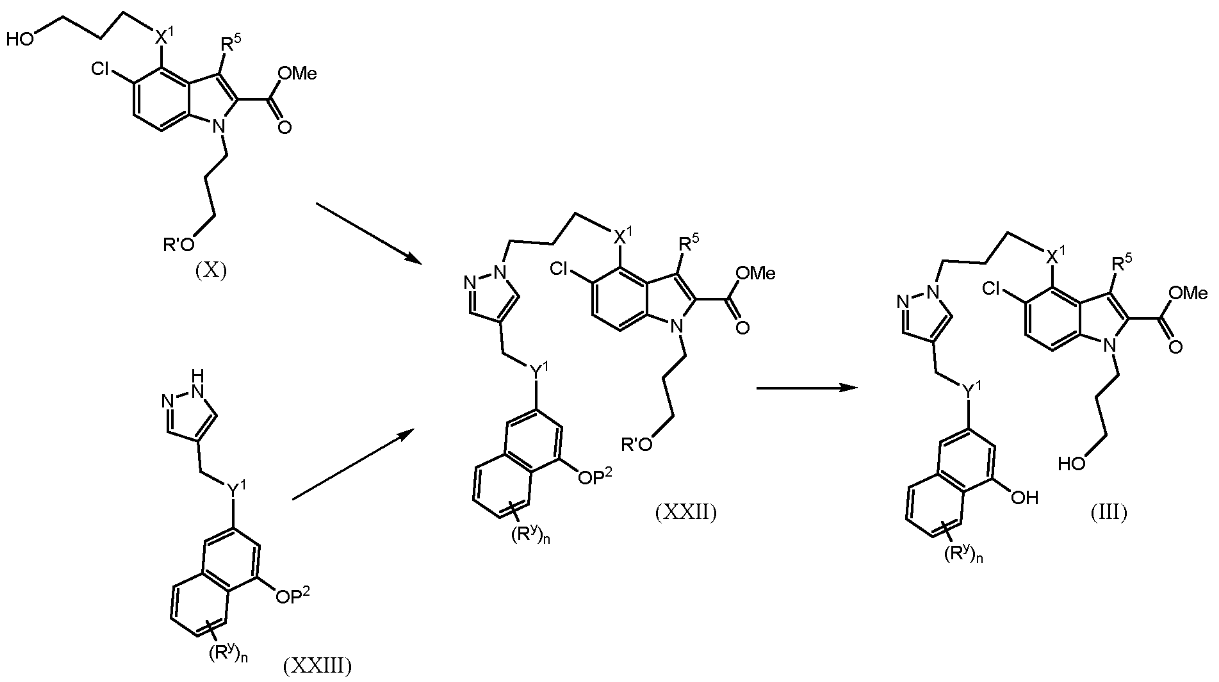

Scheme 8

- by reacting an intermediate of Formula (XXII) wherein $P^2$ and R' are suitable protecting groups such as, for example, TBDMS and THF, respectively, with a suitable deprotecting agent, such as, for example, HCl 4 M in dioxane, in a suitable solvent such as, for example, dioxane, at a suitable temperature such as, for example, room temperature.
- Intermediates of Formula (XXII) can be prepared by reacting an intermediate of Formula (X) wherein R' is a suitable protecting group such as, for example, THP, with an intermediate of Formula (XXIII), in the presence of a suitable coupling agent, such as, for example, DTBAD and triphenylphosphine, in a suitable solvent such as, for example, DCM, at a suitable temperature such as, for example, room temperature.

Alternatively, when $X^2$ represents

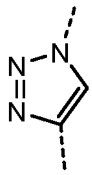
,

intermediates of Formula (III) wherein $X^1$, $R^5$, and $(R^y)_n$ are defined as in Formula (I), can be prepared according to Scheme 9,

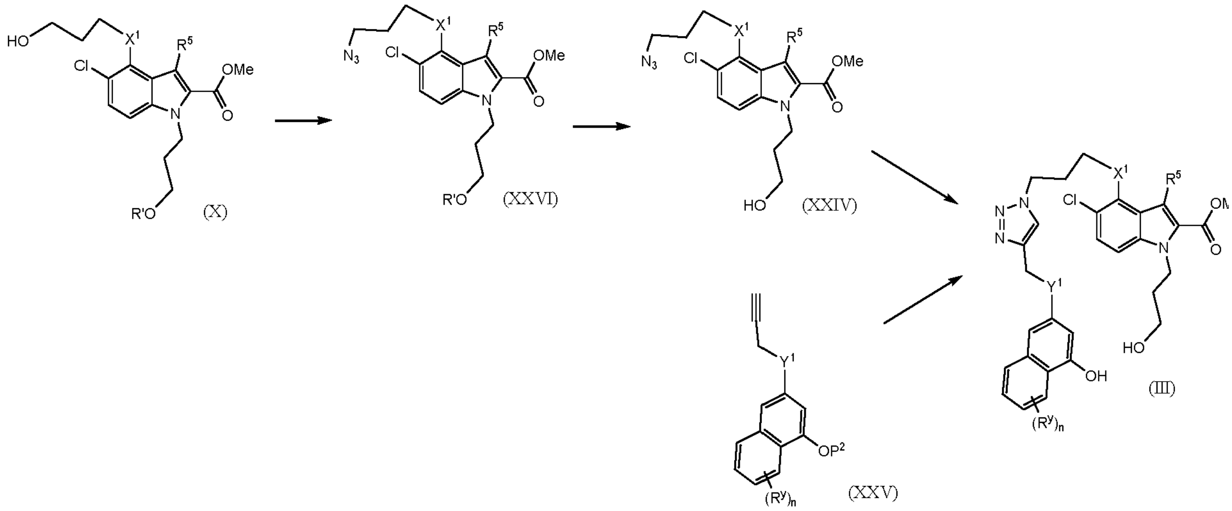
(X) (XXVI) (XXIV) (XXV) (III)

Scheme 9

- by reacting an intermediate of Formula (XXIV) with an intermediate of Formula (XXV), in the presence of a suitable catalyst such as, for example, $CuSO_4$, in the presence of a suitable additive such as, for example, sodium ascorbate, in the presence of a suitable base such as, for example, $NaHCO_3$, in a suitable solvent such as, for example, a mixture of tBuOH and water, at a suitable temperature such as, for example, room temperature.
- Intermediates of Formula (XXIV), wherein R' is TBDMS, can be prepared by reacting an intermediate of Formula (XXVI) with a suitable deprotecting agent such as, for example, TBAF, in a suitable solvent such as, for example, THF, at a suitable temperature such as, for example, room temperature.
- Intermediates of Formula (XXVI) wherein R' is TBDMS, can be prepared by reacting an intermediate of Formula (X), wherein R' is TBDMS, with a suitable azide precursor such as, for example, DPPA, in the presence of a suitable base such as, for example, DBU, in a suitable solvent such as, for example, THF, at a suitable temperature such as, for example, 60 °C.

Intermediates of Formula (XXV), wherein $Y^1$ is S (sulfur), can be prepared according to Scheme 10 by reacting an intermediate of Formula (XXVII) with a suitable propargyl precursor such as, for example, propargyl bromide, in the presence of a suitable base such as, for example, $K_2CO_3$, in a suitable solvent such as, for example, MeOH, at a suitable temperature such as, for example, room temperature.

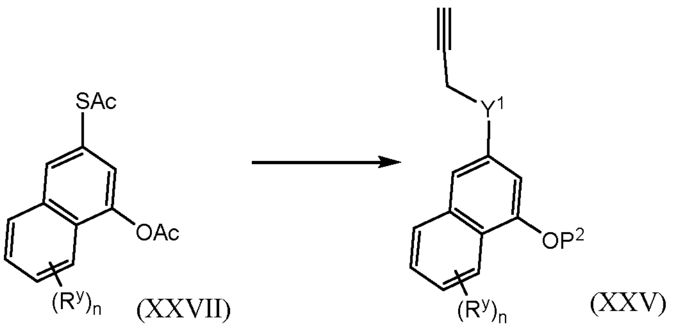

(XXVII) (XXV)

Scheme 10

Intermediates of Formula (XXIII), wherein $Y^1$ is S (sulfur) and $P^2$ is TBDMS, can be prepared according to Scheme 11,

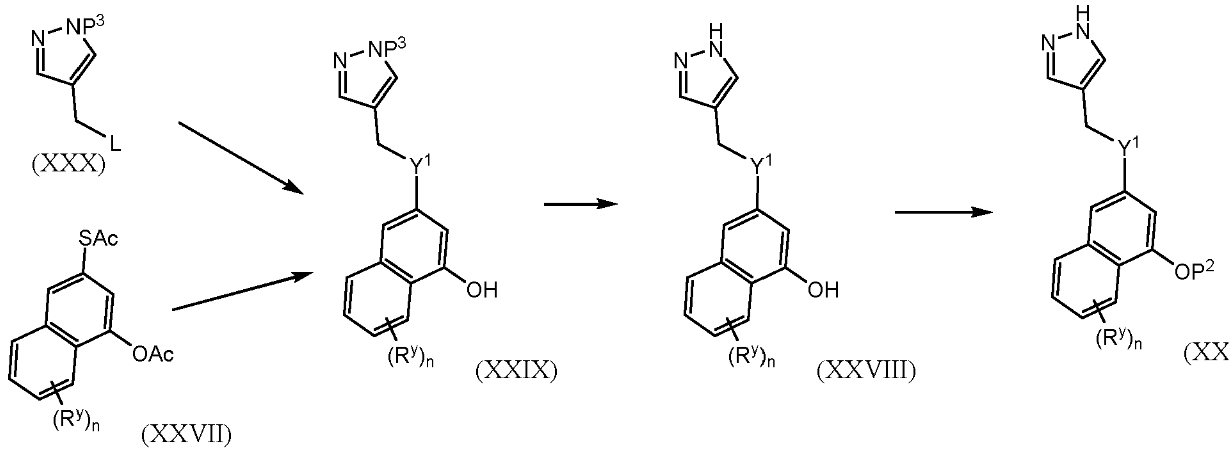

(XXX) (XXVII) (XXIX) (XXVIII) (XX

Scheme 11

- by reacting an intermediate of Formula (XXVIII) with a suitable protecting agent such as, for example, TBDMSCl, in the presence of a suitable base such as, for example, imidazole, in a suitable solvent such as, for example, DCM, at a suitable temperature such as, for example, 50 °C.

- Intermediates of Formula (XXVIII) can be prepared by reacting an intermediate of Formula (XXIX) wherein $P_3$ is a suitable protecting group such as, for example, THP, with a suitable deprotecting agent such as, for example, HCl, in a suitable solvent such as, for example, dioxane, at a suitable temperature such as, for example, room temperature.

- Intermediates of Formula (XXIX) can be prepared by reacting an intermediate of Formula (XXX), wherein $P_3$ is a suitable protecting group such as, for example, THP, and L is a suitable leaving group such as, for example, chloride, with an intermediate of Formula (XXVII), in the presence of a suitable base such as, for example, $K_2CO_3$, in the presence of a suitable catalyst such as, for example, NaI, in a suitable solvent such as, for example, MeOH, at a suitable temperature such as, for example, room temperature.

Intermediates of Formula (XXVII) can be prepared similarly to 3-(acetylthio)naphthalen-1-yl acetate (CAS [2143010-96-0]).

Intermediates of Formula (XXX) are commercially available or can prepared similarly to 4-(chloromethyl)-1-(tetrahydro-2H-pyran-2-yl)- 1H-pyrazole (CAS [2152839-94-4]).

Intermediates of Formula (XXI), wherein $X^2$ and $(R^y)_n$ are defined as in Formula (I), $P^2$ is a suitable protecting group such as, for example, TBDMS, and $Y_1$ is defined as $(CH_2)_m$ and m is defined as in Formula (I), and Hal is defined as a suitable halogen such as, for example, bromide, can be prepared according to Scheme 12,

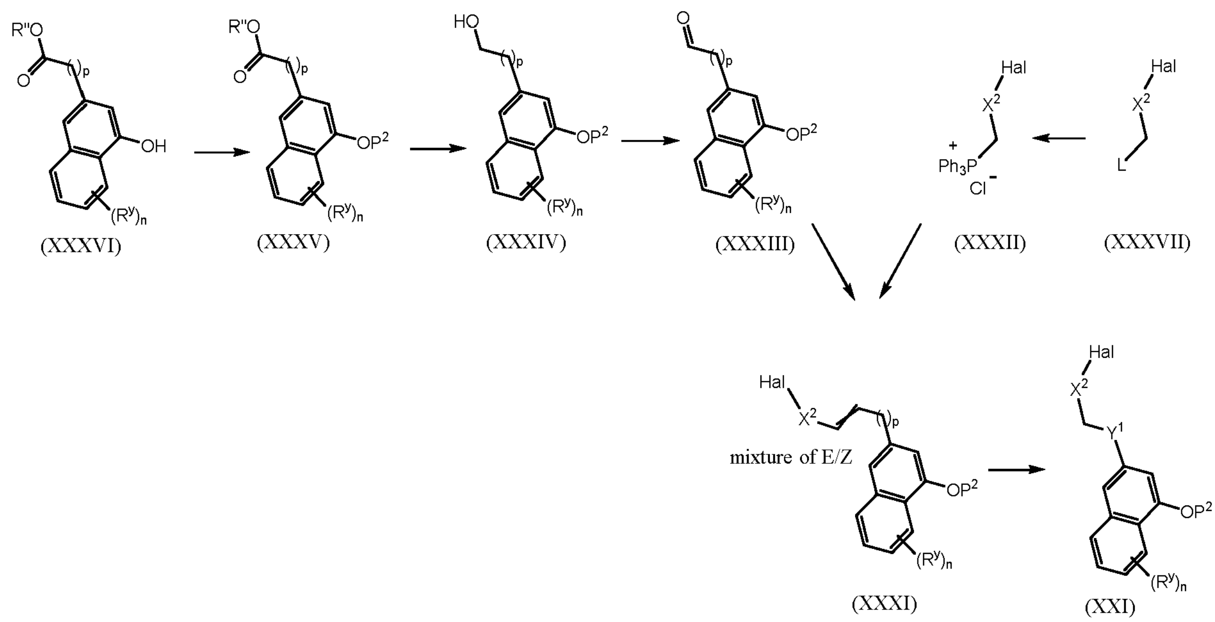


Scheme 12

- by reacting an intermediate of Formula (XXXI) with a suitable hydrogenating agent such as, for example, hydrogen, in the presence of a suitable catalyst such as, for example, $PtO_2$, in a suitable solvent such as, for example, MeOH, at a suitable temperature such as, for example, room temperature.
- Intermediates of Formula (XXXI) can be prepared by reacting an intermediate of Formula (XXXII) with an intermediate of Formula (XXXIII) in the presence of a suitable base such as, for example, NaH, in a suitable solvent such as, for example, THF, at a suitable temperature such as, for example, -20 °C or 0 °C.
- Intermediates of Formula (XXXIII), wherein p is 0 or 1, can be prepared by reacting an intermediate of Formula (XXXIV) with a suitable oxidizing agent such as, for example, $MnO_2$ or Dess-Martin periodinane, in a suitable solvent such as, for example, DCM, at a suitable temperature such as, for example, room temperature.
- Intermediates of Formula (XXXIV) can be prepared by reacting an intermediate of Formula (XXXV) with a suitable reducing agent such as, for example, $LiAlH_4$, in a suitable solvent such as, for example, THF, at a suitable temperature such as, for example, 0 °C.
- Intermediates of Formula (XXXV), wherein R" is defined as alkyl, can be prepared by reacting an intermediate of Formula (XXXVI) with a suitable protecting group such as, for example, tert-butyl(chloro)diphenylsilane (TBDPSCl), in the presence of a suitable base such as, for example, imidazole, in a suitable solvent such as, for example, DMF, at a suitable temperature such as, for example, room temperature.
- Intermediates of Formula (XXXVI) are commercially available or can be prepared similarly to methyl 7-fluoro-4-hydroxy-2-naphthoate (CAS [2092726-85-5]).
- Intermediates of Formula (XXXII) can be prepared by reacting a suitable haloheterocycle of Formula (XXXVII) such as, for example, 3-bromo-5-(chloromethyl)-1-methyl-1H-pyrazole (CAS [2109428-60-4]), with a suitable phosphine such as, for example, $PPh_3$, in a suitable solvent such as, for example, ACN, at a suitable temperature such as, for example, 85 °C.

Intermediates of Formula (XXXVII), wherein $X^2$ is defined as in Formula (I), and Hal is defined as a suitable halogen such as, for example, bromide, and L is a suitable leaving group such as, for example, chloride, can be prepared according to Scheme 13,

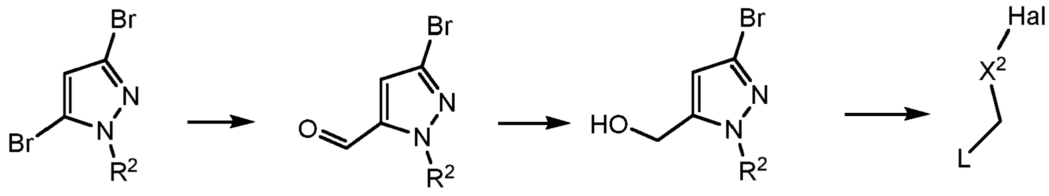

(XL) (XXXIX) (XXXVIII) (XXXVII)

Scheme 13

- by reacting an intermediate of Formula (XXXVIII) with a suitable activating agent such as, for example, thionyl chloride, in a suitable solvent such as, for example, DCM, at a suitable temperature such as, for example, room temperature.
- Intermediates of Formula (XXXVIII) can be prepared by reacting an intermediate of Formula (XXXIX) with a suitable reducing agent such as, for example, $NaBH_4$, in a suitable solvent such as, for example, MeOH, at a suitable temperature such as, for example, 15 °C.
- Intermediates of Formula (XXXIX) can be prepared by reacting an intermediate of Formula (XL) with a suitable acylating agent such as, for example, DMF, in the presence of a suitable base such as, for example, BuLi, in a suitable solvent such as, for example, THF, at a suitable temperature such as, for example, -78 °C.
- Intermediates of Formula (XL) are commercially available or can be prepared according to procedures described in literature.

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or secondary amine) of intermediates may be necessary. The need for such protection will vary depending on the nature of the remote functionality and the conditions of the preparation methods. The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

PHARMACOLOGY OF COMPOUNDS

It has been found that the compounds of the present invention inhibit one of more MCL-1 activities, such as MCL-1 antiapoptotic activity.

An MCL-1 inhibitor is a compound that blocks one or more MCL-1 functions, such as the ability to bind and repress proapoptotic effectors Bak and Bax or BH3 only sensitizers such as Bim, Noxa or Puma.

The compounds of the present invention can inhibit the MCL-1 pro-survival functions. Therefore, the compounds of the present invention may be useful in treating and / or preventing, in particular treating, diseases that are susceptible to the effects of the immune system such as cancer.

In another embodiment of the present invention, the compounds of the present invention exhibit anti-tumoral properties, for example, through immune modulation.

In an embodiment, the present invention is directed to compounds for use in methods for treating and / or preventing a cancer, wherein the cancer is selected from those described herein, comprising administering to a subject in need thereof (preferably a human), a therapeutically effective amount of a compound of Formula (I), or pharmaceutically

acceptable salt, or a solvate thereof.

In an embodiment, the present invention is directed to compounds for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of acute lymphoblastic leukemia (ALL), acute myeloid leukemia (AML), B cells acute lymphoblastic leukemia, B-cell chronic lymphocytic leukemia (CLL), bladder cancer, breast cancer, chronic lymphocytic leukemia, chronic myeloid leukemia, colon adenocarcinoma, diffuse large B cell lymphoma, esophageal cancer, follicular lymphoma, gastric cancer, head and neck cancer (including, but not limited to head and neck squamous cell carcinoma), hematopoietic cancer, hepatocellular carcinoma, Hodgkin lymphoma, liver cancer, lung cancer (including but not limited to lung adenocarcinoma), lymphoma, medulloblastoma, melanoma, monoclonal gammopathy of undetermined significance, multiple myeloma, myelodysplastic syndromes, myelofibrosis, myeloproliferative neoplasms, ovarian cancer, ovarian clear cell carcinoma, ovarian serous cystadenoma, pancreatic cancer, polycythemia vera, prostate cancer, rectum adenocarcinoma, renal cell carcinoma, smoldering multiple myeloma, T cell acute lymphoblastic leukemia, T cell lymphoma, and Waldenstroms macroglobulinemia.

In another embodiment, the present invention is directed to compounds for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is preferably selected from the group consisting of acute lymphoblastic leukemia (ALL), acute myeloid leukemia (AML), B cells acute lymphoblastic leukemia, B-cell chronic lymphocytic leukemia (CLL), breast cancer, chronic lymphocytic leukemia, chronic myeloid leukemia, diffuse large B cell lymphoma, follicular lymphoma, hematopoietic cancer, Hodgkin lymphoma, lung cancer (including, but not limited to lung adenocarcinoma) lymphoma, monoclonal gammopathy of undetermined significance, multiple myeloma, myelodysplastic syndromes, myelofibrosis, myeloproliferative neoplasms, smoldering multiple myeloma, T cell acute lymphoblastic leukemia, T cell lymphoma and Waldenstroms macroglobulinemia.

In another embodiment, the present invention is directed to compounds for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of adenocarcinoma, benign monoclonal gammopathy, biliary cancer (including, but not limited to, cholangiocarcinoma), bladder cancer, breast cancer (including, but not limited to, adenocarcinoma of the breast, papillary carcinoma of the breast, mammary cancer, medullary carcinoma of the breast), brain cancer (including, but not limited to, meningioma), glioma (including, but not limited to, astrocytoma, oligodendroglioma; medulloblastoma), bronchus cancer, cervical cancer (including, but not limited to, cervical adenocarcinoma), chordoma, choriocarcinoma, colorectal cancer (including, but not limited to, colon cancer, rectal cancer, colorectal adenocarcinoma), epithelial carcinoma, endothelial sarcoma (including, but not limited to, Kaposi's sarcoma, multiple idiopathic hemorrhagic sarcoma), endometrial cancer (including, but not limited to, uterine cancer, uterine sarcoma), esophageal cancer (including, but not limited to, adenocarcinoma of the esophagus, Barrett's adenocarinoma), Ewing sarcoma, gastric cancer (including, but not limited to, stomach adenocarcinoma), gastrointestinal stromal tumor (GIST), head and neck cancer (including, but not limited to, head and neck squamous cell carcinoma), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, kidney cancer (including, but not limited to, nephroblastoma a.k.a. Wilms' tumor, renal cell carcinoma), liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, *Lewis lung carcinoma,* lung neuroendocrine tumors, typical carcinoid, atypical carcinoid, small cell lung cancer (SCLC), and large cell neuroendocrine carcinoma),

myelodysplastic syndromes (MDS), myeloproliferative disorder (MPD), polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES), ovarian cancer (including, but not limited to, cystadenocarcinoma, ovarian embryonal carcinoma, ovarian adenocarcinoma), pancreatic cancer (including, but not limited to, pancreatic andenocarcinoma, intraductal papillary mucinous neoplasm (IPMN), Islet cell tumors), prostate cancer (including, but not limited to, prostate adenocarcinoma), skin cancer (including, but not limited to, squamous cell carcinoma (SCC), keratoacanthoma (KA), melanoma, basal cell carcinoma (BCC)) and soft tissue sarcoma (e.g. malignant fibrous histiocytoma (MFH), liposarcoma, malignant peripheral nerve sheath tumor (MPNST), chondrosarcoma, fibrosarcoma, myxosarcoma).

In another embodiment, the present invention is directed to compounds for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of benign monoclonal gammopathy, breast cancer (including, but not limited to, adenocarcinoma of the breast, papillary carcinoma of the breast, mammary cancer, medullary carcinoma of the breast), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (including, but not limited to, Waldenstrom’s macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, Lewis lung carcinoma, lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), and prostate cancer (including, but not limited to, prostate adenocarcinoma).

In another embodiment, the present invention is directed to compounds for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of prostate, lung, pancreatic, breast, ovarian, cervical, melanoma, B-cell chronic lymphocytic leukemia (CLL), acute myeloid leukemia (AML), and acute lymphoblastic leukemia (ALL).

In another embodiment, the present invention is directed to compounds for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is multiple myeloma.

The compounds according to the present invention or pharmaceutical compositions comprising said compounds, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1 and or CTLA-4 or engineered chimeric antigen receptor T cells (CART) targeting tumor associated antigens.

The compounds according to the present invention or pharmaceutical compositions comprising said compounds, may also be combined with radiotherapy or chemotherapeutic agents (including, but not limited to, anti-cancer agents) or any other pharmaceutical agent which is administered to a subject having cancer for the treatment of said subject’s cancer or for the treatment or prevention of side effects associated with the treatment of said subject’s cancer.

The compounds according to the present invention or pharmaceutical compositions comprising said compounds, may also be combined with other agents that stimulate or enhance the immune response, such as vaccines.

In an embodiment, the present invention is directed to compounds for use in methods for treating and / or preventing a cancer (wherein the cancer is selected from those described herein) comprising administering to a subject in need thereof (preferably a human), a therapeutically effective amount of co-therapy or combination therapy; wherein

the co-therapy or combination therapy comprises a compound of Formula (I) of the present invention and one or more anti-cancer agent(s) selected from the group consisting of (a) immune modulatory agent (such as inhibitors of the PD1/PDL1 immune checkpoint axis, for example antibodies (or peptides) that bind to and/or inhibit the activity of PD-1 or the activity of PD-L1 and or CTLA-4); (b) engineered chimeric antigen receptor T cells (CART) targeting tumor associated antigens; (c) radiotherapy; (d) chemotherapy; and (e) agents that stimulate or enhance the immune response, such as vaccines.

The present invention is directed to compounds of Formula (I) and pharmaceutically acceptable salts, and solvates thereof, for use as a medicament.

The present invention is directed to compounds of Formula (I) and pharmaceutically acceptable salts, and solvates thereof, for use in the inhibition of MCL-1 activity.

As used herein, unless otherwise noted, the term "anti-cancer agents" shall encompass "anti-tumor cell growth agents" and "anti-neoplastic agents".

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for treating and / or preventing diseases (preferably cancers) mentioned above.

The present invention is directed to compounds of Formula (I) and pharmaceutically acceptable salts, and solvates thereof, for treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for treating and / or preventing, in particular for treating, MCL-1 mediated diseases or conditions, preferably cancer, more preferably a cancer as herein described (for example, multiple myeloma).

The present invention is directed to compounds of Formula (I) and pharmaceutically acceptable salts, and solvates thereof, for use in treating and / or preventing, in particular for use in treating, MCL-1 mediated diseases or conditions, preferably cancer, more preferably a cancer as herein described (for example, multiple myeloma).

The present invention relates to compounds of Formula (I) and pharmaceutically acceptable salts, and solvates thereof, for the manufacture of a medicament.

The present invention relates to compounds of Formula (I) and pharmaceutically acceptable salts, and solvates thereof, for the manufacture of a medicament for the inhibition of MCL-1.

The present invention relates to compounds of Formula (I) and pharmaceutically acceptable salts, and solvates thereof, for the manufacture of a medicament for treating and / or preventing, in particular for treating, a cancer, preferably a cancer as herein described. More particularly, the cancer is a cancer which responds to inhibition of MCL-1 (for example, multiple myeloma).

The present invention is directed to compounds of Formula (I) and pharmaceutically acceptable salts, and solvates thereof, for the manufacture of a medicament for treating and / or preventing, in particular for treating, any one of the disease conditions mentioned hereinbefore.

The present invention is directed to compounds of Formula (I) and pharmaceutically acceptable salts, and solvates thereof, for the manufacture of a medicament for treating and / or preventing any one of the disease conditions mentioned hereinbefore.

The compounds of Formula (I) and pharmaceutically acceptable salts, and solvates thereof, can be administered to subjects, preferably humans, for treating and / or preventing of any one of the diseases mentioned hereinbefore.

In view of the utility of the compounds of Formula (I) and pharmaceutically acceptable salts, and solvates thereof, there is provided compounds for use in a method of treating subjects, preferably mammals such as humans, suffering from any of the diseases mentioned hereinbefore; or a method of slowing the progression of any of the diseases mentioned hereinbefore in subject, humans; or a method of preventing subjects, preferably mammals such as humans, from suffering from any one of the diseases mentioned hereinbefore.

Said methods comprise the administration, i.e. the systemic or topical administration, preferably oral or intravenous administration, more preferably oral administration, of an effective amount of a compound of Formula (I) or a pharmaceutically acceptable salt, or a solvate thereof, to subjects such as humans.

One skilled in the art will recognize that a therapeutically effective amount of the compounds of the present invention is the amount sufficient to have therapeutic activity and that this amount varies *inter alias*, depending on the type of disease, the concentration of the compound in the therapeutic formulation, and the condition of the patient. In an embodiment, a therapeutically effective daily amount may be from about 0.005 mg/kg to 100 mg/kg.

The amount of a compound according to the present invention, also referred to herein as the active ingredient, which is required to achieve a therapeutic effect may vary on case-by-case basis, for example with the specific compound,

the route of administration, the age and condition of the recipient, and the particular disorder or disease being treated. The methods of the present invention may also include administering the active ingredient on a regimen of between one and four intakes per day. In these methods of the present invention, the compounds according to the invention are preferably formulated prior to administration.

The present invention also provides compositions for treating and / or preventing the disorders (preferably a cancer as described herein) referred to herein. Said compositions comprise a therapeutically effective amount of a compound of Formula (I), or a pharmaceutically acceptable salt, or a solvate thereof, and a pharmaceutically acceptable carrier or diluent.

While it is possible for the active ingredient (e.g. a compound of the present invention) to be administered alone, it is preferable to administer it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of the present invention may be prepared by any methods well known in the art of pharmacy, for example, using methods such as those described in, for example, Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own separate pharmaceutical dosage formulation.

Therefore, in an embodiment, the present invention is directed to a product comprising, as a first active ingredient a compound according to the invention and as further, as an additional active ingredient one or more anti-cancer agent(s), as a combined preparation for simultaneous, separate or sequential use in the treatment of patients suffering from cancer.

The one or more other anti-cancer agents and the compound according to the present invention may be administered simultaneously (e.g. in separate or unitary compositions) or sequentially, in either order. In an embodiment, the two or more compounds are administered within a period and / or in an amount and / or a manner that is sufficient to ensure that an advantageous or synergistic effect is achieved. It will be appreciated that the preferred method and order of administration and the respective dosage amounts and regimes for each component of the combination will depend on the particular other anti-cancer agent and the compound of the present invention being administered, their route of administration, the particular condition, in particular tumor, being treated and the particular host being treated.

The following examples further illustrate the present invention.

## EXAMPLES

Several methods for preparing the Compounds of this invention are illustrated in the following examples. Unless otherwise noted, all starting materials were obtained from commercial suppliers and used without further purification, or alternatively can be synthesized by a skilled person by using well-known methods.

| Abbreviation | Meaning |
|---|---|
| **ACN** | acetonitrile |
| **DBU** | 1,8-diazabicyclo(5.4.0)undec-7-ene |
| **DCM** | dichloromethane |
| **DIPE** | diisopropyl ether |
| **DIPEA** | *N,N*-diisopropylethylamine |
| **DMF** | *N*,*N*-dimethylformamide |
| **DPPA** | diphenylphosphoryl azide |
| **DTBAD** | di-tert-butyl azodicarboxylate |
| **$Et_3N$ or TEA** | trietylamine |
| **EtOAc** | ethyl acetate |
| **EtOH** | ethanol |

(continued)

| Abbreviation | Meaning |
|---|---|
| **FA** | formic acid |
| **h** | hour(s) |
| **HPLC** | high performance liquid chromatography |
| **iPrNH$_2$** | isopropylamine |
| **MeOH** | methanol |
| **min** | minute(s) |
| **MsCl** | methanesulfonyl chloride |
| **BuLi** | butyllithium |
| **NBS** | N-Bromosuccinimide |
| **Pd/C** | palladium on carbon |
| **PPh$_3$** | triphenylphosphine |
| **SFC** | supercritical fluid chromatography |
| **Pd(AmPhos)$_2$Cl$_2$** | bis(di-tert-butyl(4-dimethylaminophenyl)phosphine)dichloropalladium(II) |
| **TBAF** | tetrabutylammonium fluoride |
| **RP** | reversed phase |
| **THF** | tetrahydrofuran |
| **TBDPSCl** | tert-butyl(chloro)diphenylsilane |
| **TBDMSCl** | tert-butyldimethylsilyl chloride |

As understood by a person skilled in the art, Compounds synthesized using the protocols as indicated may contain residual solvent or minor impurities.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

**Preparation of intermediates**

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

**Intermediate 1**

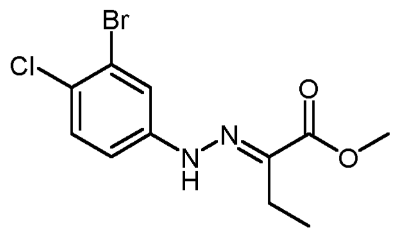

A solution of (3-bromo-4-chlorophenyl)hydrazine (CAS [1172589-41-1]) (4.66 g, 18.05 mmol) and methyl 2-oxobutanoate (CAS [3952-66-7]) (1.02 eq.) in HCl (93 mL, 1.25 M in MeOH) was refluxed for 90 min. The reaction was cooled to room temperature and volatiles were removed under reduced pressure to give 5.77 g of Intermediate 1 as a

brown oily residue that solidified within minutes, and was used as such in the following step.

## Intermediate 2

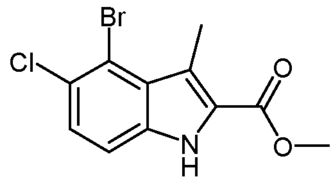

A suspension of Intermediate 1 (5.77 g, crude) in acetic acid (37 mL) was heated to 70 °C. Sulfuric acid (4.81 mL, 5 eq.) was added dropwise over 10 min (exotherm developed and a precipitate formed). After 15 additional min, the reaction mixture was cooled to room temperature and then to 0 °C by adding ice. The solid precipitate was filtered and washed with water until the filtrate was of neutral pH. The crude reaction mixture was triturated with cold heptane/diisopropylether (8/2, 50 mL) to give an off-white solid. This solid was purified by preparative SFC (Stationary phase: Chiralpak Daicel IG 20 x 250 mm, Mobile phase: $CO_2$, EtOH + 0.4 % $iPrNH_2$) to give Intermediate 2 (1.75 g, 32 %).

## Intermediate 3

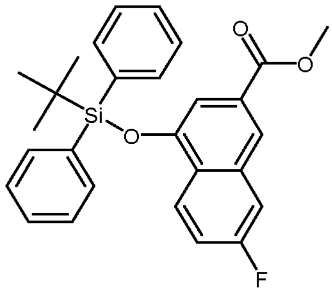

TBDPSCl (14.66 g, 1.5 eq.) was added to a solution of methyl 7-fluoro-4-hydroxy-2-naphthoate (CAS [2092726-85-5]) (8 g, 35.56 mmol) and imidazole (7.26 g, 3 eq.) in DCM (500 mL), cooled to 0 °C under nitrogen atmosphere. Once the addition was complete, the reaction mixture was stirred at room temperature overnight. The reaction was quenched by addition of water (100 mL). The mixture was extracted with EtOAc (3 x 200 mL). The combined organic layer was dried over $Na_2SO_4$, filtered, and concentrated to afford a yellow oil. This oil was purified by flash column chromatography on silica gel (petroleum ether/EtOAc - 1:0 to 1:1) to afford Intermediate 3 (14 g, yield: 86 %) as a yellow oil.

## Intermediate 4

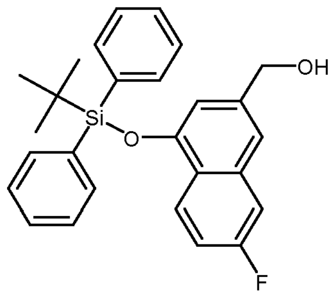

$LiAlH_4$ (1.39 g, 1.2 eq.) was added slowly to a solution of Intermediate 3 (14 g, 30.53 mmol) in THF (200 mL), cooled to 0 °C under nitrogen atmosphere. Once the addition was complete, the reaction mixture was stirred at 0 °C for 2 h. The reaction was quenched by slow addition of water (2 mL) followed by a 10 % aqueous NaOH solution (2 mL) at 0 °C. The heterogeneous mixture was filtered, and the filter cake was washed with DCM (200 mL). The filtrate was evaporated and the residue was purified by flash column chromatography on silica gel (petroleum ether/EtOAc - 1:0 to 1:1) to give Intermediate 4 (12 g, yield: 90 %) as a yellow solid.

## Intermediate 5

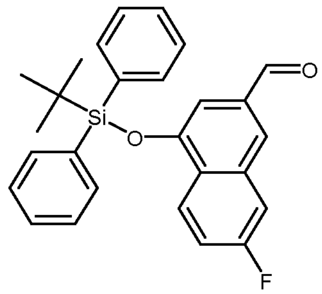

$MnO_2$ (29.1 g, 12 eq.) was added to a solution of Intermediate 4 (12 g, 27.87 mmol) in DCM (200 mL) at room temperature. The resulting solution was stirred at room temperature overnight. The reaction mixture was filtered and the filtrate was concentrated. The residue was purified by flash column chromatography over silica gel (eluent: petroleum ether/EtOAc, 100/0 to 50/50) to afford Intermediate 5 (12 g, yield: 99 %) as a yellow oil.

## Intermediate 6

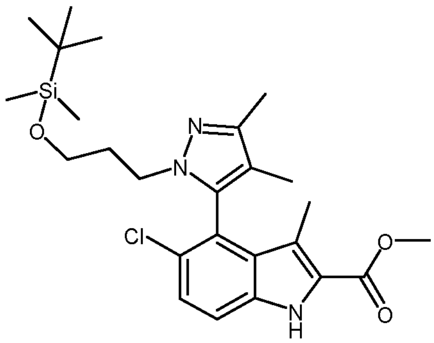

$Pd(AmPhos)_2Cl_2$ (CAS [887919-35-9]) (1.17 g, 0.05 eq.) was added to a mixture of Intermediate 2 (10 g, 33 mmol), Intermediate 29 (21 g, 1.2 eq.) and $K_2CO_3$ (6.85 g, 1.5 eq.) in 1,4-dioxane (240 mL) and $H_2O$ (40 mL) under nitrogen atmosphere and the reaction mixture was stirred for 6 h at 110 °C. The solution was cooled down to room temperature and filtered through a Celite® pad. The filtrate was evaporated and the residue was diluted with EtOAc (250 mL) and $H_2O$ (50 mL). The separated aqueous layer was extracted with EtOAc (60 mL) and the combined organic layer was washed with brine (100 mL), dried over $MgSO_4$, filtered, and concentrated under vacuum to afford Intermediate 6 as a brown liquid (65 % pure), which was used without further purification.

## Intermediate 7

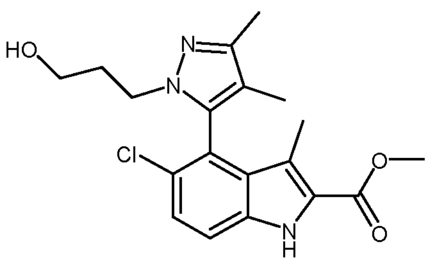

HCl (4 M in 1,4-dioxane, 191 mL, 10 eq.) was added to a solution of Intermediate 6 (57.6 g, 65 % pure, 76.4 mmol) in 1,4-dioxane (160 mL) and the reaction mixture was stirred at room temperature for 10 min. The mixture was concentrated under vacuum and the residue was diluted with EtOAc (300 mL). The mixture was washed with saturated aqueous NaHCOs (2 x 100 mL) and the aqueous layer was extracted with EtOAc (100 mL). The combined organic layer was washed with brine (150 mL), dried over $MgSO_4$, filtered, and concentrated under vacuum. The residue was purified by flash column chromatography over silica gel (370 g, gradient: petroleum ether/EtOAc 100/0 to 0/100) to afford Intermediate 7 (22.2 g, yield: 60 %) as a yellow gum.

**Intermediate 8**

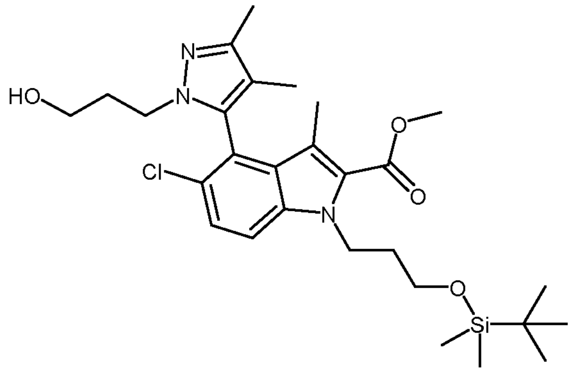

A mixture of Intermediate 7 (22.18 g, 52.58 mmol), (3-bromopropoxy)-tert-butyldimethylsilane (14.65 g, 1.1 eq.) and $K_2CO_3$ (14.53 g, 2 eq.) in ACN (250 mL) was stirred at 80 °C for 16 h. The reaction mixture was cooled down and filtered. The filter cake was rinsed with ACN (3 x 100 mL). The combined filtrate was concentrated under vacuum. The residue was purified by flash column chromatography over silica gel (340 g, gradient: petroleum ether/EtOAc 100/0 to 50/50) to afford Intermediate 8 (8.52 g, yield: 26 %) as a yellow gum that solidified on standing.

**Intermediate 9**

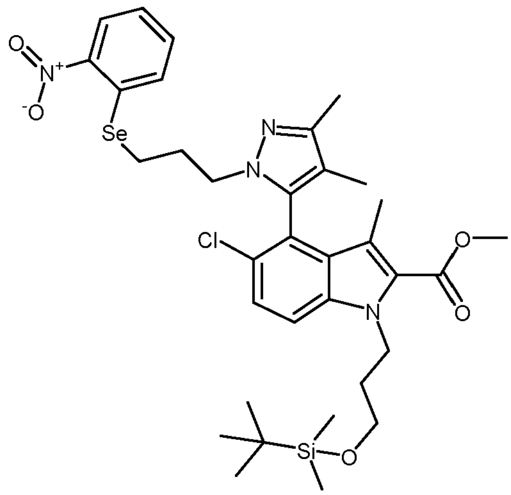

Tri-n-butylphosphine (2.9 mL, 2.4 eq.) was added to a solution of Intermediate 8 (3 g, 4.79 mmol) and 2-nitrophenyl selenocyanate (2.18 g, 2 eq.) in THF (50 mL) at 0 °C, under nitrogen atmosphere. The reaction mixture was stirred at room temperature for 2 h. The solvent was evaporated and the residue was purified by flash column chromatography over silica gel (40 g, eluent: petroleum ether/EtOAc 100/0 to 50/50) to afford Intermediate 9 (3 g, yield: 81 %) as a brown liquid.

## Intermediate 10

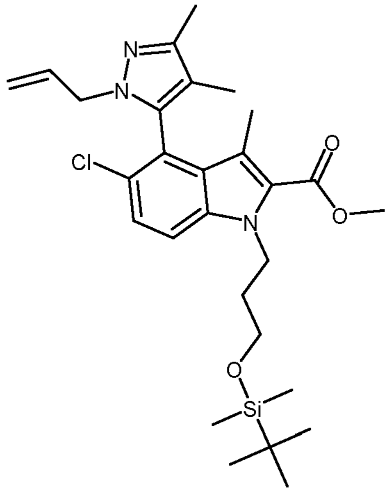

$H_2O_2$ (1.9 mL, 5 eq.) was added to a solution of Intermediate 9 (3 g, 3.87 mmol) in THF (40 mL) and the reaction mixture was stirred at room temperature overnight. The reaction mixture was diluted with saturated aqueous $Na_2S_2O_3$ (4 mL) and water (10 mL) and it was extracted with EtOAc (10 mL). The combined organic layer was washed with brine (15 mL), dried over $MgSO_4$, filtered, and evaporated. The residue was purified by flash column chromatography over silica gel (40 g, eluent: petroleum ether/EtOAc 100/0 to 85/15) to give Intermediate 10 (1.94 g, yield: 91 %) as a brown liquid.

## Intermediate 11

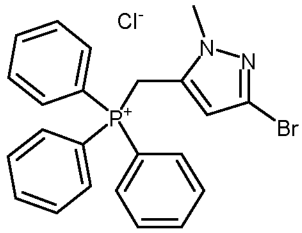

$PPh_3$ (260 mg, 1.2 eq.) was added to a solution of 3-bromo-5-(chloromethyl)-1-methyl-1H-pyrazole (CAS [2109428-60-4]) (173 mg, 0.82 mmol) in ACN (2 mL) and the reaction mixture was stirred at 85 °C for 24 h. The solvent was evaporated and the residue was triturated with petroleum ether (20 mL) and was stirred at room temperature for 1 h. The solid was filtered, collected, and dried under vacuum to afford Intermediate 11 (262 mg, yield: 61 %) as a white solid.

**Intermediate 12 and Intermediate 13**

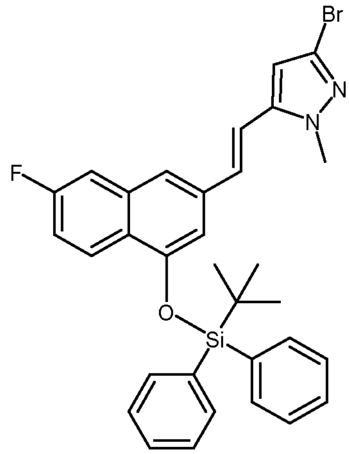

Intermediate 12

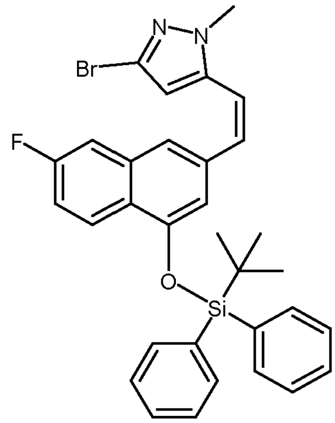

Intermediate 13

NaH (60 % in mineral oil, 30 mg, 1.5 eq.) was added to a solution of Intermediate 11 (260 mg, 0.5 mmol) in THF (3 mL) at 0 °C and the reaction mixture was stirred at the same temperature for 1 h. The mixture was cooled down to -20 °C and Intermediate 5 was added. The reaction mixture was stirred at -20 °C for 2 h. The reaction was quenched by addition of water (20 mL) and the mixture was extracted with DCM (3 × 20 mL). The combined organic layer was dried with $Na_2SO_4$, filtered, and evaporated. The residue was purified by column chromatography on silica gel (eluent: petroleum ether/EtOAc 100/0 to 85/15) to afford Intermediate 12 (120 mg, yield: 41 %) and Intermediate 13 (110 mg, yield: 37 %), both as white solids.

## Intermediate 14

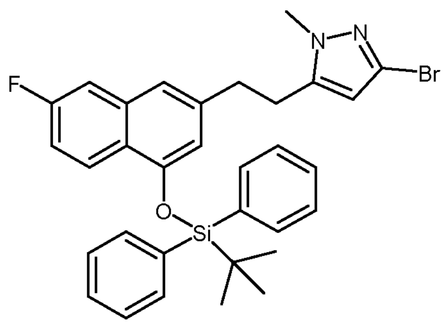

$PtO_2$ (155 mg, 0.4 eq.) was added to a solution of Intermediate 13 (1 g, 1.7 mmol) in MeOH (15 mL) and the reaction mixture was stirred under hydrogen atmosphere (2 eq.) at room temperature for 24 h. The reaction mixture was filtered and the filtrate was evaporated to afford Intermediate 14 (1 g, yield: 59 %), crude and used without further purification.

## Intermediate 15

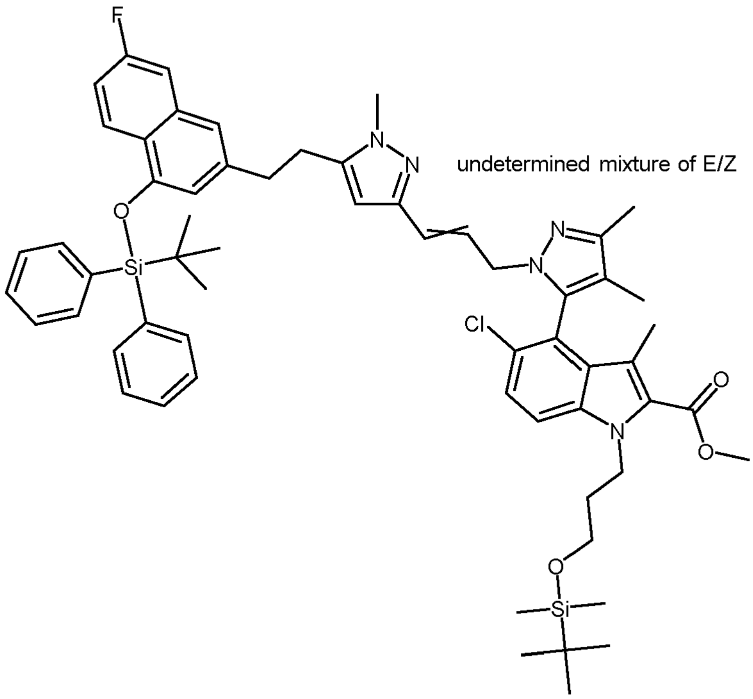


DIPEA (481 µL, 3 eq.) was added to a solution of Intermediate 10 (500 mg, 0.943 mmol), Intermediate 14 (831 mg, 1.5 eq.), bis(tri-tert-butylphosphine)palladium (CAS [53199-31-8]) (96 mg, 0.2 eq.) in DMF (10 mL) under nitrogen atmosphere and the reaction mixture was stirred at 120 °C for 3 h. The reaction mixture was diluted with EtOAc (100 mL) and water (50 mL). The organic layer was separated and washed with brine (2 × 35 mL). The combined aqueous layer was back-extracted with EtOAc (50 mL). The combined organic layer was dried over $MgSO_4$, filtered, and concentrated under vacuum. The residue was purified by flash chromatography on silica gel (80 g, gradient: DCM/MeOH 100/0 to 95/5) to afford Intermediate 15 (723 mg, yield: 74 %) as a foam.

## Intermediate 16

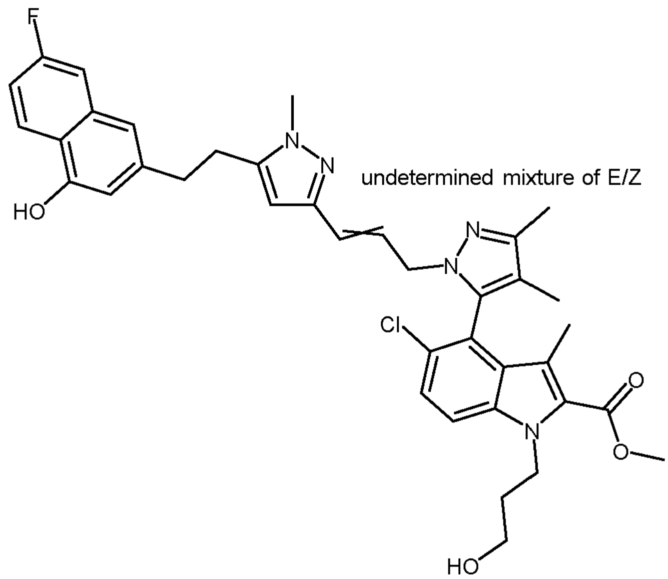


TBAF (1 M in THF, 2 mL, 2.5 eq.) was added to a solution of Intermediate 15 (865 mg, 0.834 mmol) in dry THF (10 mL) under nitrogen atmosphere and the reaction mixture was stirred at room temperature for 3 h. The solvent was evaporated and the residue was dissolved in EtOAc (100 mL). The mixture was washed with water (50 mL) and brine (50 mL). The combined aqueous layer was back-extracted with EtOAc (50 mL). The combined organic layer was evaporated. The residue was purified by flash column chromatography on silica gel (80 g, gradient: DCM/MeOH 100/0 to 95/5) to afford Intermediate 16 (337 mg, yield: 59 %) as a white foam.

**Intermediate 17**

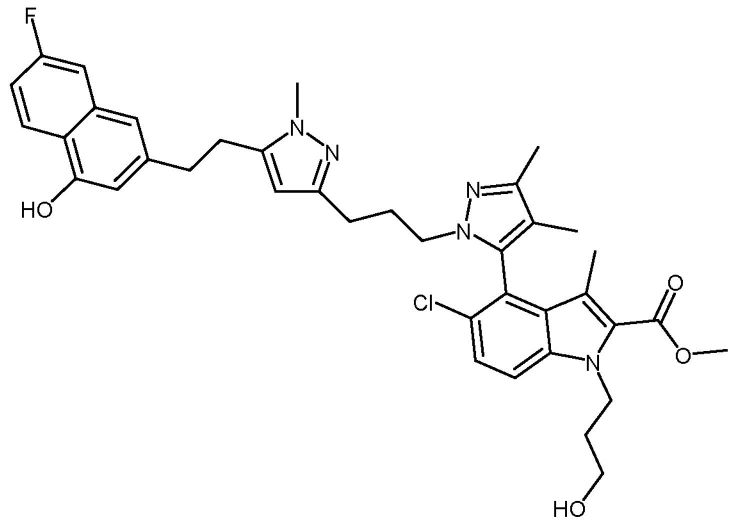

A solution of Intermediate 16 (337 mg, 0.493 mmol) in EtOAc (21 mL) was stirred under hydrogen atmosphere (1 atm) in the presence of Pd/C (10 %, 52 mg, 0.1 eq.) at room temperature for 16 h. The reaction mixture was filtered over Dicalite®, the filter cake was washed with EtOAc and the filtrate was evaporated to afford Intermediate 17, used directly in the next step.

**Intermediate 18 and Intermediate 19**

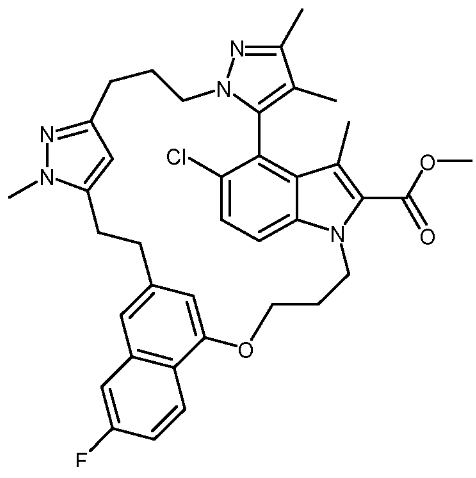

Intermediate 18 (Ra or Sa; one atropisomer but absolute stereochemistry undetermined) Intermediate 19 (Sa or Ra; one atropisomer but absolute stereochemistry undetermined) A solution of Intermediate 17 (338 mg, 0.493 mmol) and DTBAD (227 mg, 2 eq.) in toluene (10 mL) and THF (2 mL) was added with a syringe pump (0.1 mL/min) to a solution of $PPh_3$ (258 mg, 2 eq.) in toluene (10 mL), stirring at 70 °C. Once the addition was complete, the reaction mixture was allowed to cool down to room temperature and the solvents were evaporated. The residue was purified by flash column chromatography on silica gel (80 g, gradient: EtOAc/MeOH 100/0 to 95/5). The obtained product was purified by preparative SFC (Stationary phase: Chiralpak Diacel AD 20 × 250 mm, Mobile phase: $CO_2$, EtOH + 0.4 % $iPrNH_2$) to afford Intermediate 18 (86 mg, yield: 26 %) and Intermediate 19 (88 mg, yield: 27 %) as foams.

## Intermediate 20

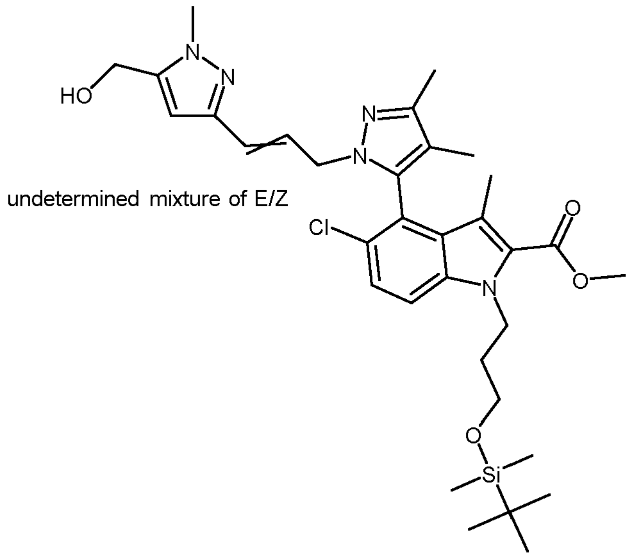


Intermediate 20 was prepared according to an analogous procedure as for Intermediate 15, starting from Intermediate 10 and (3-bromo-1-methyl-1H-pyrazol-5-yl)methanol (CAS [1784533-05-6]) instead of Intermediate 14.

## Intermediate 21

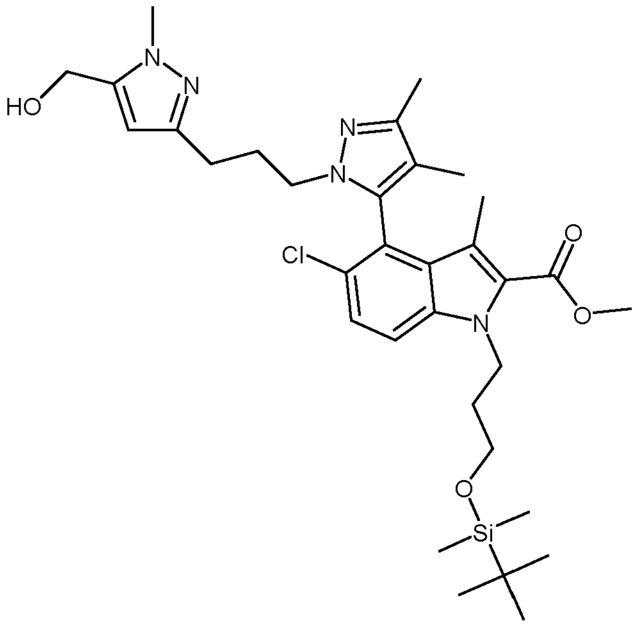

Intermediate 21 was prepared according to an analogous procedure as for Intermediate 17, starting from Intermediate 20 instead of Intermediate 16.

**Intermediate 22**

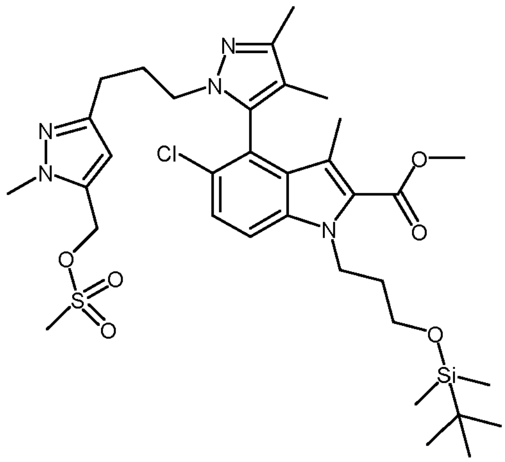

MsCl (141 μL, 2.5 eq.) was added dropwise to a solution of Intermediate 21 (467 mg, 0.727 mmol) and $Et_3N$ (303 μL, 3 eq.) in DCM (16 mL), stirring at 0 °C under nitrogen atmosphere. The reaction mixture was warmed up to room temperature and was stirred for 1 h. The reaction mixture was diluted with DCM (25 mL) and treated with saturated aqueous NaHCOs (20 mL). The layers were separated and the aqueous one was extracted with DCM (25 mL). The combined organic layer was dried over $MgSO_4$, filtered, and evaporated to afford Intermediate 22, used directly in the next step.

**Intermediate 23**

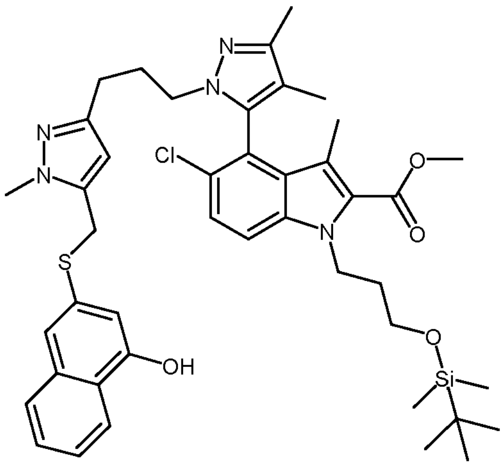

Intermediate 23

$K_2CO_3$ (151 mg, 1.5 eq.) was added to a solution of ethanethioic acid, S-[4-(acetyloxy)-2-naphthalenyl] ester (CAS [2143010-96-0]) (246 mg, 1.3 eq.) in degassed MeOH (7.5 mL). After 5 min, a solution of Intermediate 22 (524 mg, 0.727 mmol) in THF (3.7 mL) was added and the reaction mixture was stirred at room temperature for 1 h. Additional $K_2CO_3$ (151 mg, 1.5 eq.) was added and stirring was continued at room temperature for 30 min. The solvent was evaporated and the residue was dissolved in EtOAc (50 mL) and water (30 mL). The layers were separated and the aqueous one was extracted with EtOAc (50 mL). The combined organic layer was dried over $MgSO_4$, filtered, and evaporated. The residue was purified by flash column chromatography on silica gel (80 g, gradient: DCM/MeOH($NH_3$) 100/0 to 95/5) to afford Intermediate 23 (220 mg, yield: 38 %) as a foam.

## Intermediate 24

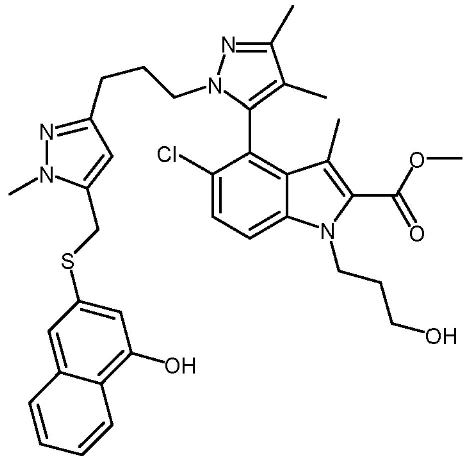

TBAF (1 M in THF, 0.36 mL, 1.3 eq.) was added to a solution of Intermediate 23 (220 mg, 0.275 mmol) in dry THF (5 mL) under nitrogen atmosphere and the reaction mixture was stirred at room temperature for 3 h. The solvent was evaporated and the residue was dissolved in EtOAc (30 mL). The mixture was washed with water (20 mL) and brine (20 mL). The combined aqueous layer was back-extracted with EtOAc (50 mL). The organic layer was evaporated and the residue was purified by flash column chromatography on silica gel (24 g, gradient: DCM/MeOH 100/0 to 95/5) to afford Intermediate 24 (188 mg, yield: 99 %) as a yellow foam.

**Intermediate 25 and Intermediate 26**

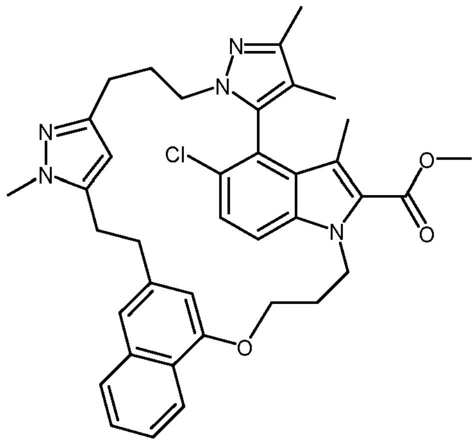

Intermediate 25 (Ra or Sa; one atropisomer but absolute stereochemistry undetermined) Intermediate 26 (Sa or Ra; one atropisomer but absolute stereochemistry undetermined) A solution of Intermediate 24 (188 mg, 0.274 mmol) and DTBAD (252 mg, 4 eq.) in toluene (6 mL) and THF (1 mL) was added with a syringe pump (0.1 mL/min) to a solution of $PPh_3$ (287 mg, 4 eq.) in toluene (6 mL), stirring at 70 °C. Once the addition was complete, the reaction mixture was allowed to cool down to room temperature and the solvents were evaporated. The residue was purified by flash column chromatography on silica gel (40 g, gradient: DCM/MeOH 100/0 to 95/5). The obtained product was separated into its atropisomers by preparative SFC (Stationary phase: Daicel Chiralpak OJ 20 × 250 mm, Mobile phase: $CO_2$, EtOH + 0.4 % $iPrNH_2$) to afford Intermediate 25 (33 mg, yield: 18 %) and Intermediate 26 (33 mg, yield: 18 %).

**Intermediate 27a and Intermediate 27b**

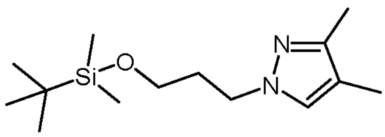

Intermediate 27a

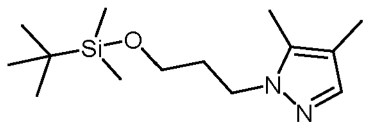

Intermediate 27b

NaH (60 % in mineral oil, 8.73g, 1.05 eq.) was added portionwise to a stirred solution of 3,4-dimethyl-1H-pyrazole (CAS [2820-37-3]) (20 g, 208 mmol) and (3-bromopropoxy)-tert-butyldimethylsilane (CAS [89031-84-5]) (55.32 g, 1.05 eq.) in DMF (400 mL) at 0 °C. The reaction mixture was allowed to warm up to room temperature and was stirred for 30 min. The reaction was quenched by addition of saturated aqueous $NH_4$ Cl (200 mL) and water (200 mL). The mixture was extracted with EtOAc (3 × 200 mL). The combined organic layer was washed with water (400 mL) and brine (300 mL), dried over $MgSO_4$, filtered, and concentrated under reduced pressure to afford a mixture of Intermediate 27a and Intermediate 27b (63.2 g, yield: 56 %) as a yellow liquid.

## Intermediate 28

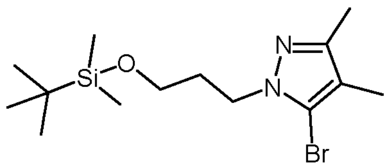

NBS (44 g, 2.1 eq.) was added to a solution of the mixture of Intermediate 27a and Intermediate 27b (63.2 g, 117.7 mmol) in DCM (600 mL) at 0 °C under nitrogen atmosphere. The reaction mixture was allowed to warm up to room temperature and was stirred for 1 h. The reaction mixture was diluted carefully with saturated aqueous $Na_2SO_3$ (200 mL). The yellow solution was stirred at room temperature for 10 min. The organic layer was separated, dried over $MgSO_4$, filtered, and concentrated under reduced pressure. The residue was purified by flash column chromatography on silica gel (330 g, gradient: petroleum ether/EtOAc 100/0 to 90/10) to give a yellow liquid that was further purified by preparative HPLC (Stationary phase: RP Xtimate Prep C18 OBD - 5 μm, 40 × 150 mm, Mobile phase: water (10 mM $NH_4HCO_3$)/$CH_3CN$ 10/90 to 3/97). The obtained product was diluted with water (70 mL) and EtOAc (150 mL). The organic layer was separated, dried over $MgSO_4$, filtered, and concentrated under reduced pressure to give Intermediate 28 (24 g, yield: 59 %) as a yellow liquid.

## Intermediate 29

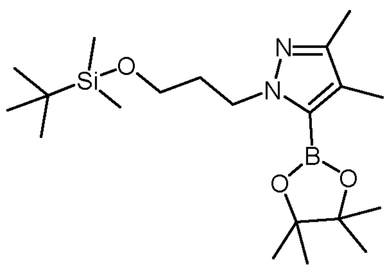

BuLi (2.5 M in hexane, 6.33 mL, 1.1 eq.) was added dropwise to a solution of Intermediate 28 (5 g, 14.39 mmol) in dry THF (75 mL) at -78 °C, under nitrogen atmosphere. The reaction mixture was stirred at -78 °C for 45 min, before the dropwise addition of 2-isopropoxy-4,4,5,5-tetramethyl-1,3,2-dioxaborolane (CAS [61676-62-8]) (3.52 mL, 1.2 eq.). The reaction mixture was allowed to warm up to room temperature and was stirred for 16 h. The reaction was quenched by addition of saturated aqueous $NH_4Cl$ (25 mL). The reaction mixture was diluted with water (50 mL) and EtOAc (100 mL) and the layers were separated. The aqueous layer was back-extracted with EtOAc (75 mL). The combined organic layer was dried over $MgSO_4$, filtered, and evaporated. The residue was purified by flash column chromatography on silica gel (120 g, gradient: heptane/EtOAc 100/0 to 80/20) to afford Intermediate 29 (4.19 g, yield: 74 %) as a colourless oil.

## Intermediate 30

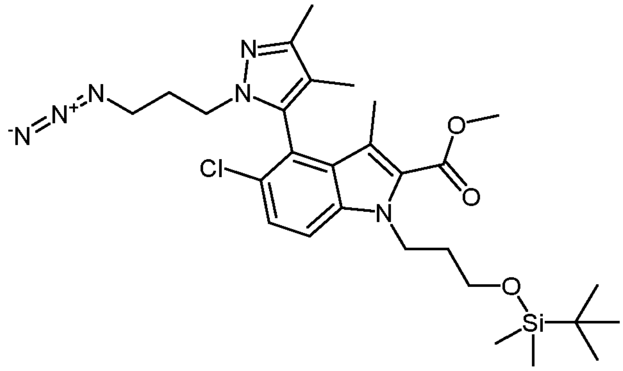

DPPA (CAS [26386-88-9], 774 mg, 2.812 mmol, 2 eq.) and DBU (642 mg, 4.219 mmol, 3 eq.) were added to a solution of Intermediate 8 (800 mg, 1.406 mmol) in dry THF (16 mL) under nitrogen atmosphere. The reaction mixture was stirred at 60 °C overnight. Water (20 mL) was added and the mixture was extracted with EtOAc (20 mL × 3). The combined organic layer was washed with brine (10 mL), dried over $Na_2SO_4$, filtered, and evaporated to give Intermediate 30 (1.25 g, yield: 91 %) as a yellow oil, used without further purification.

## Intermediate 31

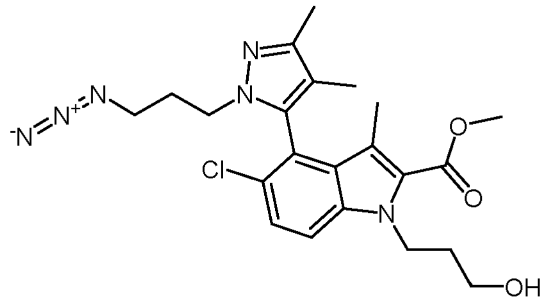

TBAF (1 M in THF, 3.271 mL, 3.271 mmol, 2.5 eq.) was added to a solution of Intermediate 30 (1.25 g, 1.287 mmol) in dry THF (20 mL). The reaction mixture was stirred at room temperature for 2 h. Water (20 mL) was added and the mixture was extracted with EtOAc (30 mL × 2). The combined organic layer was washed with brine (20 mL), dried over $Na_2SO_4$, filtered, and evaporated. The residue was purified by flash column chromatography over silica gel (eluent: petroleum ether/EtOAc from 100/0 to 30/70) to give Intermediate 31 (540 mg, yield: 91 %) as a yellow oil.

## Intermediate 32

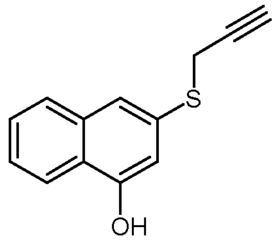

Propargyl bromide (600 mg, 4.034 mmol, 1.05 eq.) and $K_2CO_3$ (1327 mg, 9.604 mmol, 2.5 eq.) were added to a solution of 3-(acetylthio)naphthalen-1-yl acetate (CAS [2143010-96-0], 1 g, 3.842 mmol) in MeOH (20 mL). The reaction mixture was stirred at room temperature for 2 h. Water (30 mL) was added and the mixture was extracted with EtOAc (50 mL × 2). The combined organic layer was washed with brine (30 mL), dried over $Na_2SO_4$, filtered, and evaporated. The residue was purified by flash column chromatography over silica gel (eluent: petroleum ether/EtOAc from 100/0 to 70/30) to give Intermediate 32 (600 mg, yield: 72 %) as a brown solid.

## Intermediate 33

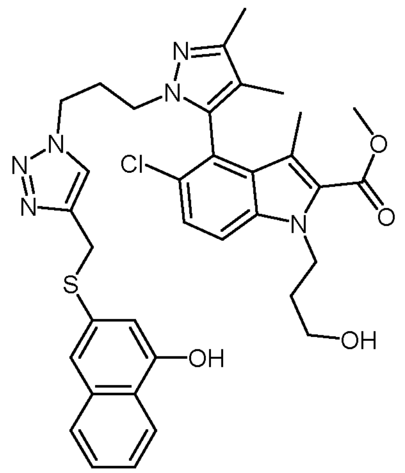

Intermediate 32 (306 mg, 1.412 mmol, 1.2 eq.), sodium ascorbate (93 mg, 0.471 mmol, 0.4 eq.), NaHCOs (296 mg, 3.53 mmol, 3 eq.), and $CuSO_4$ (38 mg, 0.235 mmol, 0.2 eq.) were added to a solution of Intermediate 31 (540 mg, 1.177 mmol) in tBuOH (8 mL) and water (8 mL). The reaction mixture was stirred at room temperature overnight. Water (20 mL) and EtOAc (20 mL) were added. The mixture was separated and the aqueous layer was extracted with EtOAc (20 mL × 2). The combined organic layer was washed with brine (20 mL), dried over $Na_2SO_4$, filtered, and evaporated. The residue was purified by flash column chromatography over silica gel (eluent: petroleum ether/EtOAc from 100/0 to 0/100, followed by EtOAc/MeOH from 100/0 to 70/30) to give Intermediate 33 (500 mg, yield: 63 %) as a brown solid.

**Intermediate 34 and Intermediate 35**

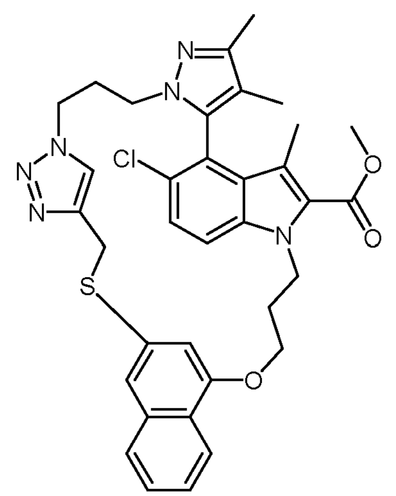

Intermediate 34: Ra or Sa; one atropisomer but absolute stereochemistry undetermined Intermediate 35: Sa or Ra; one atropisomer but absolute stereochemistry undetermined Triphenylphosphine (351 mg, 1.337 mmol, 3 eq.) and DTBAD (308 mg, 1.337 mmol, 3 eq.) were added to a solution of Intermediate 33 (300 mg, 0.446 mmol) in dry DCM (12 mL) at room temperature under nitrogen atmosphere. The reaction mixture was stirred at room temperature overnight. The solvent was evaporated and the residue was purified by high-performance liquid chromatography (Column: Welch Xtimate C18 150 × 40 mm × 10 um; water/ACN 25/75 to 0/100) followed by SFC (column: Daicel Chiralcel OD (250 mm × 30 mm, 10 um); $CO_2$/EtOH (0.1 % $NH_3.H_2O$) 30/70 to 60/40) to give Intermediate 34 (100 mg, yield: 34 %) and Intermediate 35 (100 mg, yield: 34 %).

Intermediate 36

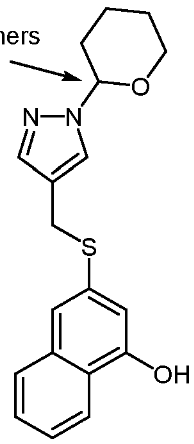


$K_2CO_3$ (8.265 g, 59.8 mmol, 3 eq.) and NaI (4.482 g, 29.9 mmol, 1.5 eq.) were added to a solution of 4-(chloromethyl)-1-(tetrahydro-2H-pyran-2-yl)-1H-pyrazole (CAS [2152839-94-4], 4 g, 19.9 mmol) and 3-(acetylthio)naphthalen-1-yl acetate (CAS [2143010-96-0], 6.828 g, 25.9 mmol, 1.3 eq.) in MeOH (120 mL). The reaction mixture was stirred at room temperature for 16 h. The solvent was evaporated and water (800 mL) was added. The pH was adjusted to 6 using aqueous HCl (1 N). The mixture was extracted with EtOAc (200 mL × 2). The combined organic layer was dried with $Na_2SO_4$, filtered, and evaporated. The residue was purified by flash column chromatography over silica gel (eluent: petroleum ether/EtOAc from 100/0 to 60/40) to afford Intermediate 36 (4.5 g, 75 % pure, yield: 50 %) as a yellow oil.

Intermediate 37

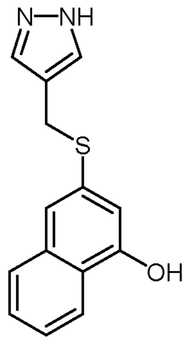

HCl (4 M in dioxane, 20 mL, 80 mmol, 8 eq.) was added to a solution of Intermediate 36 (4.5 g, 75 % pure, 9.989 mmol) in dioxane (40 mL). The reaction mixture was stirred at room temperature for 16 h. The solvent was evaporated and saturated aqueous $NaHCO_3$ was added (pH 7). The mixture was extracted with EtOAc (200 mL × 2). The combined organic layer was dried with $Na_2SO_4$, filtered, and evaporated. The residue was purified by flash column chromatography over silica gel (eluent: petroleum ether/EtOAc from 100/0 to 40/60) to afford Intermediate 37 (2.8 g, 75 % pure, yield: 82 %) as a yellow oil.

Intermediate 38

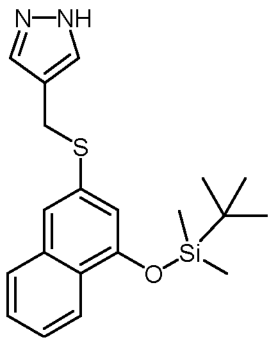

TBDMSCl (1.842 g, 12.224 mmol, 1.5 eq.) and imidazole (1.387 g, 20.374 mmol, 2.5 eq.) were added to a solution of Intermediate 37 (2.8 g, 75 % pure, 8.15 mmol) in DCM (60 mL). The reaction mixture was stirred at 50 °C for 16 h. The reaction was quenched by addition of water (150 mL) and the mixture was extracted with DCM (80 mL × 2). The combined organic layer was dried with $Na_2SO_4$, filtered, and evaporated. The residue was purified by flash column chromatography over silica gel (eluent: petroleum ether/EtOAc from 100/0 to 70/30) to afford Intermediate 38 (3.5 g, 76 % pure) as a yellow oil.

**Intermediate 39**

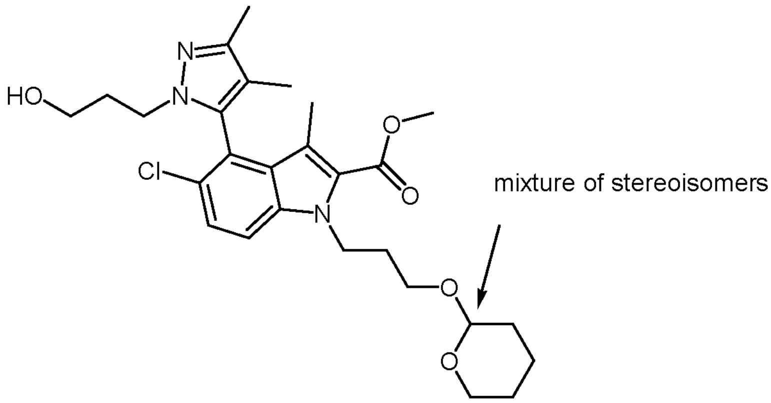


A mixture of Intermediate 7 (4 g, 68 % pure, 7.25 mmol), 2-(3-bromopropoxy)tetrahydro-2H-pyran (CAS [33821-94-2], 2.426 g, 10.875 mmol, 1.5 eq.), and $K_2CO_3$ (2.505 g, 18.125 mmol, 2.5 eq.) in ACN (40 mL) was stirred at 85 °C for 16 h. After cooling, the reaction mixture was filtered and the filter cake was rinsed with ACN (3 × 30 mL). The combined organic layer was evaporated and the residue was purified by flash column chromatography (40 g silica gel, petroleum ether/EtOAc 100/0 to 30/70) to give Intermediate 39 (3.7 g, yield: 90 %) as a yellow gum.

**Intermediate 40**

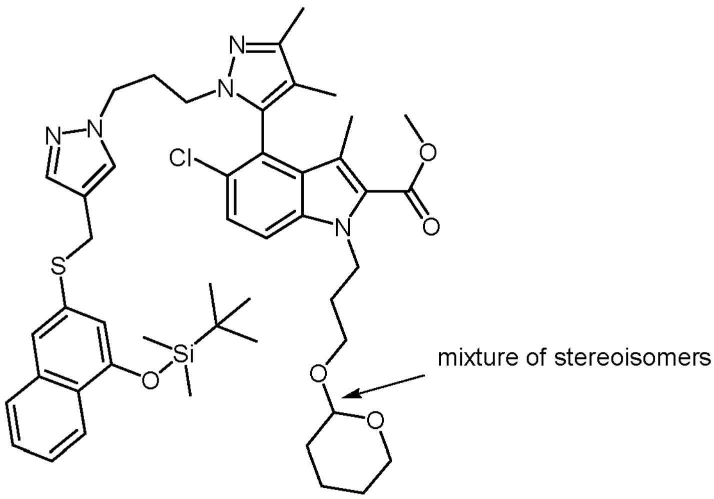


Triphenylphosphine (1.39 g, 5.299 mmol, 3 eq.) was added to a solution of Intermediate 39 (1 g, 1.766 mmol) and Intermediate 38 (1.03 g, 2.12 mmol, 1.2 eq.) in DCM (40 mL) and the mixture was purged with nitrogen three times. DTBAD (1.22 g, 5.299 mmol, 3 eq.) was added to the reaction mixture at room temperature and the mixture was stirred at room temperature for 16 h. The solvent was evaporated and the residue was purified by flash column chromatography over silica gel (eluent: petroleum ether/EtOAc from 100/0 to 55/45) to afford Intermediate 40, still impure and used without further purification (1 g, 26 % pure, yield: 17 %) as a yellow oil.

## Intermediate 41

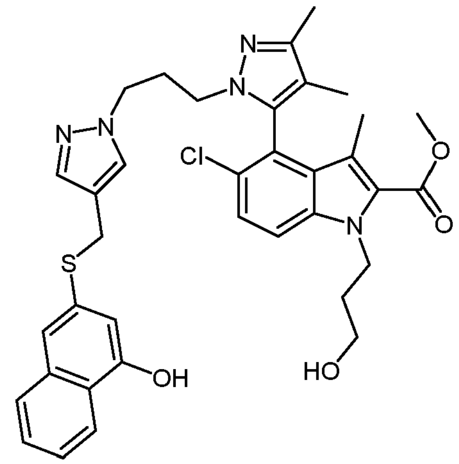

HCl (4 M in dioxane, 4 mL, 16 mmol, 53 eq.) was added to a solution of Intermediate 40 (1 g, 26 % pure, 0.302 mmol) in MeOH (20 mL). The reaction mixture was stirred at room temperature for 16 h. The solvent was evaporated and saturated aqueous NaHCOs was added. The mixture was extracted with EtOAc (50 mL × 2). The organic layer was evaporated and the residue was purified by flash column chromatography over silica gel (eluent: petroleum ether/EtOAc from 100/0 to 0/100), followed by preparative high-performance liquid chromatography (column: Phenomenex Gemini NX-C18 (75 × 30 mm × 3 um); water (0.05 % $NH_3.H_2O$ + 10 mM $NH_4HCO_3$)/ACN 45/55 to 15/85) to afford Intermediate 41 (100 mg, yield: 48 %).

**Intermediate 42 and Intermediate 43**

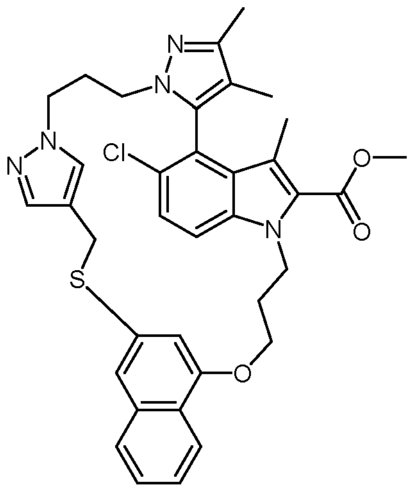

Intermediate 42: Sa or Ra; one atropisomer but absolute stereochemistry undetermined Intermediate 43: Ra or Sa; one atropisomer but absolute stereochemistry undetermined Triphenylphosphine (79 mg, 0.391 mmol, 3 eq.) was added to a solution of Intermediate 41 (90 mg, 0.13 mmol) and 1,1'-(azodicarbonyl)dipiperidine (CAS [10465-81-3], 99 mg, 0.391 mmol, 3 eq.) in DCM (10 mL) at room temperature and the mixture was purged with nitrogen. The reaction mixture was stirred at room temperature for 16 h. The solvent was evaporated and the residue was purified by preparative high-performance liquid chromatography (Column: Phenomenex Luna C18 75 × 30 mm × 3 um; water (0.2 % FA)/ACN 35/65 to 5/95) to give the racemic mixture of Intermediate 42 and Intermediate 43. This mixture was separated into its atropisomers by SFC (Daicel Chiralpak AD-H (250 mm × 30 mm, 5 um); $CO_2$/EtOH (0.1 % $NH_3.H_2O$) 55/45) to afford Intermediate 42 (30 mg, yield: 34 %) and Intermediate 43 (30 mg, yield: 34 %).

**Preparation of Compounds**

Compound 1

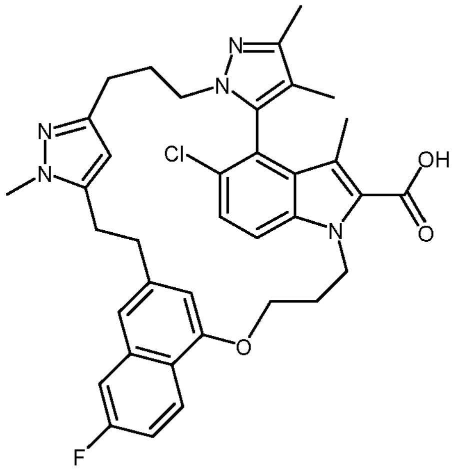

Ra or Sa; one atropisomer but absolute stereochemistry undetermined A solution of LiOH (46 mg, 15 eq.) in water (0.45 mL) was added to a solution of Intermediate 18 (86 mg, 0.129 mmol) in THF/MeOH (0.9 mL/0.9 mL). The reaction mixture was stirred at 60 °C for 3 h. After cooling, the reaction mixture was diluted with MeOH and directly injected into preparative HPLC (Stationary phase: RP XBridge Prep C18 OBD - 10 μm, 30 × 150 mm, Mobile phase: 0.25 % $NH_4HCO_3$ solution in water, $CH_3CN$). The obtained product was triturated in DIPE, filtered, and dried at 50 °C under vacuum to afford Compound 1 (70 mg, yield: 83 %).

$^1H$ NMR (400 MHz, DMSO-$d_6$) δ ppm 1.27 - 1.37 (m, 1 H), 1.63 (s, 3 H), 1.64 - 1.73 (m, 1 H), 1.81 (s, 3 H), 2.07 - 2.16 (m, 5 H), 2.29 - 2.39 (m, 2 H), 2.77 - 2.89 (m, 2 H), 2.94 - 3.05 (m, 2 H), 3.31 - 3.44 (m, 2 H), 3.46 (s, 3 H), 3.72 - 3.86 (m, 2 H), 4.57 - 4.68 (m, 1 H), 4.80 (s, 1 H), 5.05 - 5.16 (m, 1 H), 6.37 (s, 1 H), 7.02 (s, 1 H), 7.21 - 7.29 (m, 2 H), 7.42 (dd, J=10.5, 2.6 Hz, 1 H), 7.78 (d, J=9.0 Hz, 1 H), 8.12 (dd, J=9.2, 5.9 Hz, 1 H).

Compound 2

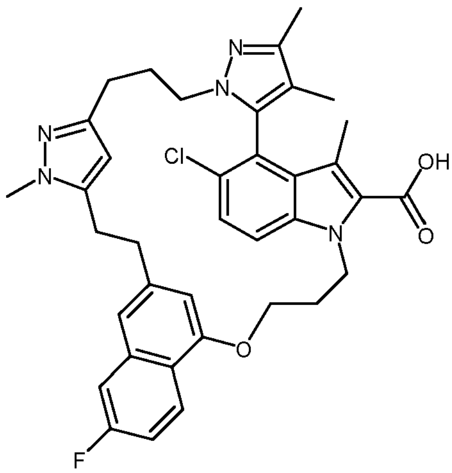

Sa or Ra; one atropisomer but absolute stereochemistry undetermined Compound 2 was prepared according to an analogous procedure as for Compound 1, starting from Intermediate 19 instead of Intermediate 18.

$^1H$ NMR (400 MHz, DMSO-$d_6$) δ ppm 1.27 - 1.37 (m, 1 H), 1.63 (s, 3 H), 1.64 - 1.72 (m, 1 H), 1.81 (s, 3 H), 2.07 - 2.16 (m, 5 H), 2.29 - 2.38 (m, 2 H), 2.77 - 2.88 (m, 2 H), 2.94 - 3.05 (m, 2 H), 3.31 - 3.44 (m, 2 H), 3.46 (s, 3 H), 3.72 - 3.86 (m, 2 H), 4.57 - 4.69 (m, 1 H), 4.80 (s, 1 H), 5.05 - 5.16 (m, 1 H), 6.37 (s, 1 H), 7.02 (s, 1 H), 7.21 - 7.29 (m, 2 H), 7.42 (dd, J=10.5, 2.6 Hz, 1 H), 7.78 (d, J=9.0 Hz, 1 H), 8.12 (dd, J=9.2, 5.9 Hz, 1 H).

## Compound 3

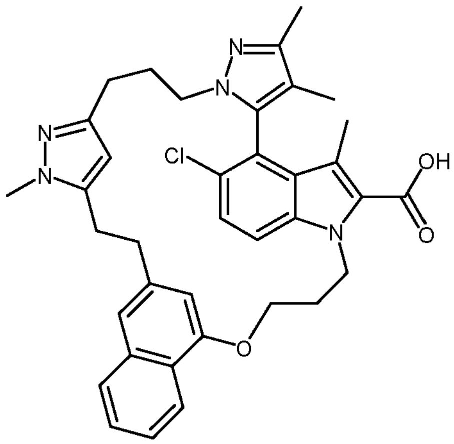

Ra or Sa; one atropisomer but absolute stereochemistry undetermined A solution of LiOH (18 mg, 15 eq.) in water (0.6 mL) was added to a solution of Intermediate 25 (33 mg, 0.049 mmol) in THF/MeOH (1.2 mL/1.2 mL). The reaction mixture was stirred at 60 °C for 3 h. After cooling, the reaction mixture was diluted with MeOH and directly injected into preparative HPLC (Stationary phase: RP XBridge Prep C18 OBD - 10 μm, 30 × 150 mm, Mobile phase: 0.25 % $NH_4HCO_3$ solution in water, $CH_3CN$) to afford Compound 3 (27 mg, yield: 83 %) as a white solid.

$^1H$ NMR (400 MHz, DMSO-$d_6$) δ ppm 1.13 - 1.25 (m, 1 H), 1.62 (s, 3 H), 1.64 - 1.73 (m, 1 H), 1.80 (s, 3 H), 2.01 - 2.16 (m, 5 H), 2.36 - 2.46 (m, 2 H), 3.18 - 3.24 (m, 2 H), 3.69 (s, 3 H), 3.93 - 4.06 (m, 2 H), 4.12 (d, J=15.4 Hz, 1 H), 4.24 (d, J=15.4 Hz, 1 H), 4.57 - 4.69 (m, 2 H), 5.10 (dt, J=14.6, 5.0 Hz, 1 H), 6.74 (s, 1 H), 7.17 (s, 1 H), 7.30 (d, J=9.0 Hz, 1 H), 7.38 - 7.48 (m, 2 H), 7.62 - 7.66 (m, 1 H), 7.88 (d, J=9.0 Hz, 1 H), 7.98 - 8.03 (m, 1 H).

## Compound 4

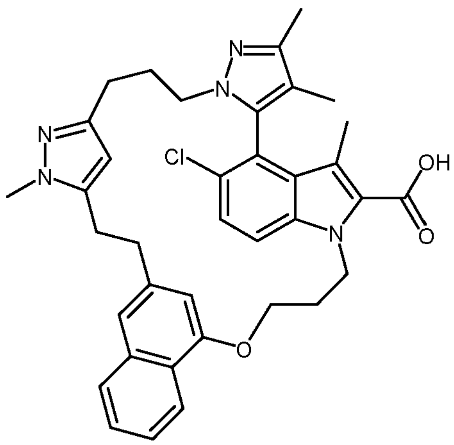

Sa or Ra; one atropisomer but absolute stereochemistry undetermined Compound 4 was prepared according to an analogous procedure as for Compound 3, starting from Intermediate 26 instead of Intermediate 25.

$^1H$ NMR (400 MHz, DMSO-$d_6$) δ ppm 1.12 - 1.24 (m, 1 H), 1.62 (s, 3 H), 1.64 - 1.74 (m, 1 H), 1.80 (s, 3 H), 2.01 - 2.15 (m, 5 H), 2.36 - 2.45 (m, 2 H), 3.17 - 3.24 (m, 2 H), 3.69 (s, 3 H), 3.92 - 4.06 (m, 2 H), 4.12 (d, J=15.4 Hz, 1 H), 4.22 (d, J=15.4 Hz, 1 H), 4.58 - 4.68 (m, 2 H), 5.10 (dt, J=14.6, 5.1 Hz, 1 H), 6.73 (d, J=1.1 Hz, 1 H), 7.17 (s, 1 H), 7.31 (d, J=9.0 Hz, 1 H), 7.38 - 7.49 (m, 2 H), 7.62 - 7.67 (m, 1 H), 7.89 (d, J=9.0 Hz, 1 H), 7.98 - 8.03 (m, 1 H).

## Compound 5

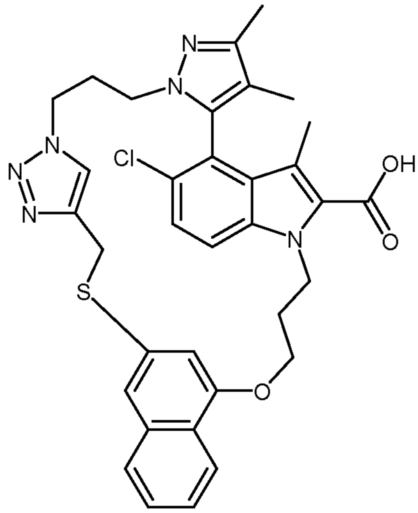

Ra or Sa; one atropisomer but absolute stereochemistry undetermined $LiOH.H_2O$ (17 mg, 0.41 mmol, 3 eq.) was added to a solution of Intermediate 34 (90 mg, 0.137 mmol) in THF (1.5 mL), MeOH (0.5 mL), and water (0.5 mL). The reaction mixture was stirred at 50 °C for 3 h. The pH of the mixture was adjusted to 5 with aqueous HCl (1 N) and it was extracted with EtOAc (15 mL). The organic layer was washed with brine (5 mL), dried over $Na_2SO_4$, filtered, and evaporated to give Compound 5 (70 mg, yield: 79 %) as a white solid.

$^1H$ NMR (400 MHz, DMSO-t/6) d ppm = 8.15 (d, J=8.0 Hz, 1H), 7.70 (d, J=8.8 Hz, 2H), 7.53 - 7.42 (m, 2H), 7.22 (s, 1H), 7.15 (s, 1H), 7.06 (s, 1H), 6.99 (d, J=9.0 Hz, 1H), 5.08 - 4.97 (m, 1H), 4.60 (ddd, J=5.0, 8.9, 14.2 Hz, 1H), 4.23 (s, 2H), 4.17 - 3.95 (m, 4H), 3.45 - 3.41 (m, 2H), 2.42 (br s, 2H), 2.14 (s, 3H), 2.03 - 1.84 (m, 2H), 1.80 (s, 3H), 1.62 (s, 3H)

## Compound 6

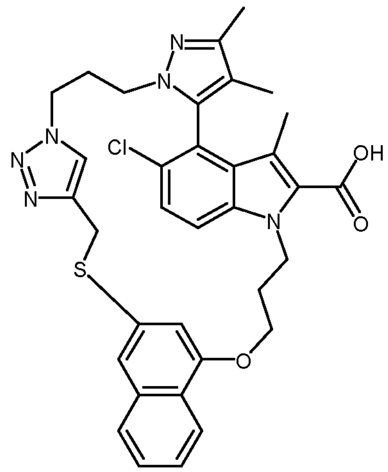

Sa or Ra; one atropisomer but absolute stereochemistry undetermined Compound 6 was prepared according to an analogous procedure as for Compound 5, starting from Intermediate 35 instead of Intermediate 34.

$^1H$ NMR (400 MHz, DMSO-*d*6) d ppm = 8.15 (d, J=8.0 Hz, 1H), 7.70 (d, J=9.0 Hz, 2H), 7.53 - 7.41 (m, 2H), 7.22 (s, 1H), 7.16 (s, 1H), 7.06 (s, 1H), 6.99 (d, J=9.0 Hz, 1H), 5.09 - 4.97 (m, 1H), 4.66 - 4.53 (m, 1H), 4.23 (s, 2H), 4.18 - 3.95 (m, 4H), 3.47 - 3.38 (m, 2H), 2.42 (br s, 2H), 2.15 (s, 3H), 2.03 - 1.84 (m, 2H), 1.80 (s, 3H), 1.62 (s, 3H)

## Compound 7

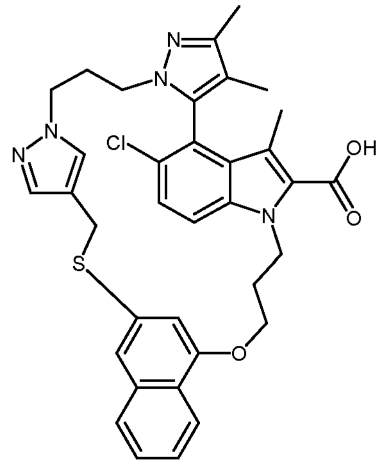

Ra or Sa; one atropisomer but absolute stereochemistry undetermined LiOH (53 mg, 2.229 mmol, 50 eq.) was added to a solution of Intermediate 43 (30 mg, 0.044 mmol) in MeOH (3 mL) and water (1 mL. The reaction mixture was stirred at room temperature for 32 h. The solvent was evaporated and the residue was purified by preparative high-performance liquid chromatography (Column: Phenomenex Luna C18 75 × 30 mm × 3 um; water (0.2 % FA)/ACN 36/64 to 6/94) to afford Compound 7 (26 mg, yield: 91 %).

$^1$H NMR (400 MHz, MeOH-d4) d ppm = 8.11 (br d, J=7.7 Hz, 1H), 7.65 - 7.54 (m, 2H), 7.44 (dquin, J=1.5, 7.1 Hz, 2H), 7.20 (s, 1H), 7.13 (s, 1H), 7.04 (d, J=8.8 Hz, 1H), 6.60 (d, J=1.1 Hz, 1H), 6.52 (s, 1H), 5.17 (td, J=4.2, 14.4 Hz, 1H), 4.63 (ddd, J=4.3, 9.9, 14.3 Hz, 1H), 4.12 - 4.00 (m, 1H), 3.96 - 3.89 (m, 1H), 3.88 - 3.76 (m, 4H), 3.16 - 3.05 (m, 1H), 2.99 - 2.87 (m, 1H), 2.58 - 2.34 (m, 2H), 2.21 (s, 3H), 2.15 (br d, J=6.6 Hz, 1H), 1.83 (s, 3H), 1.67 (s, 3H), 1.65 - 1.58 (m, 1H)

## Compound 8

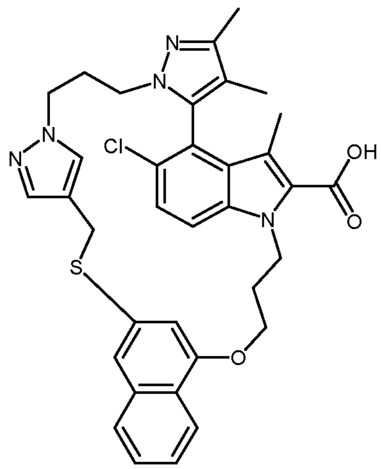

Sa or Ra; one atropisomer but absolute stereochemistry undetermined Compound 8 was prepared according to an analogous procedure as for Compound 7, starting from Intermediate 42 instead of Intermediate 43.

$^1$H NMR (400 MHz, MeOH-*d*4) d ppm = 8.12 (br d, J=7.5 Hz, 1H), 7.61 (d, J=8.8 Hz, 2H), 7.49 - 7.39 (m, 2H), 7.24 - 7.11 (m, 2H), 7.04 (d, J=8.8 Hz, 1H), 6.65 - 6.46 (m, 2H), 5.17 (br d, J=14.6 Hz, 1H), 4.63 (br t, J=9.8 Hz, 1H), 4.12 - 4.02 (m, 1H), 3.99 - 3.91 (m, 1H), 3.90 - 3.75 (m, 4H), 3.22 - 3.08 (m, 1H), 3.05 - 2.91 (m, 1H), 2.46 (br s, 2H), 2.21 (s, 3H), 2.19 - 2.09 (m, 1H), 1.84 (s, 3H), 1.68 (s, 3H), 1.67 - 1.58 (m, 1H)

**Analytical Analysis**

LCMS methods

The High Performance Liquid Chromatography (HPLC) measurement was performed using a LC pump, a diode-array (DAD) or a UV detector and a column as specified in the respective methods. If necessary, additional

detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method Codes (Flow expressed in mL/min; column temperature (T) in °C; Run time in minutes)

| Method Code | Instrument | column | mobile phase | gradient | Flow<br>Col T | Run time |
|---|---|---|---|---|---|---|
| 1 | Waters: Acquity® UPLC® - DAD and SQD | Waters: BEH (1.8 µm, 2.1*100 mm) | A: 10mM $CH_3COONH_4$ in 95 % $H_2O$ +5% $CH_3CN$<br>B: $CH_3CN$ | From 100 % A to 5 % A in 2.10 min, to 0 % A in 0.90 min, to 5 % A in 0.5 min | 0.7<br>55 | 3.5 |
| 2 | Agilent 1200 equiped with MSD 6110 or equivalent | Waters Xbridge-C18 column (5 µm, 2.0 × 50 mm) | A: water with 0.04 % TFA<br>B: $CH_3CN$ + 0.02 % TFA | 90 % A hold for 0.8 min<br>Gradient to 20 % A and 80 % B in 3.7 min.<br>Hold for 3 min.<br>Return to 90 % A in 2 min and hold for 0.5 min. | 0.8<br>50 | 10 |
| 3 | Agilent 1200 equiped with MSD 6110 | Waters Xbridge-C18 column (5 µm, 2.1 × 50 mm) | A: water with % TFA 0.04<br>B: $CH_3CN$ + 0.02 % TFA | 70 % A hold for 0.8 min<br>Gradient to 10 % A and 90 % B in 3.7 min.<br>Hold for 3 min.<br>Return to 70 % A in 2 min and hold for 0.5 min. | 0.8<br>50 | 10 |

| Compound number | LCMS results |
|---|---|
| 1 | confirms the MW (RT: 1.79, [M+H]+ 654, Method: 1) |
| 2 | confirms the MW (RT: 1.79, [M+H]+ 654, Method: 1 |
| 3 | confirms the MW (RT: 1.83, [M+H]+ 654, Method: 1) |
| 4 | confirms the MW (RT: 1.82, [M+H]+ 654, Method: 1) |
| 5 | confirms the MW (RT: 3.97, [M+H]+ 641, Method: 2) |
| 6 | confirms the MW (RT: 3.97, [M+H]+ 641, Method: 2) |
| 7 | confirms the MW (RT: 3.33, [M+H]+ 640, Method: 3) |
| 8 | confirms the MW (RT: 3.33, [M+H]+ 640, Method: 3) |

SFC methods:

The SFC measurement was performed using an Analytical Supercritical fluid chromatography (SFC) system composed by a binary pump for delivering carbon dioxide (CO2) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Analytical SFC-MS Methods (Flow expressed in mL/min; column temperature (Col T) in °C; Run time in minutes, Backpressure (BPR) in bars. "$iPrNH_2$" means isopropylamine, "iPrOH" means 2-propanol, "EtOH" means ethanol, "min" mean minutes.

SFC methods:

| Method code | column | mobile phase | gradient | Flow | Run time |
|---|---|---|---|---|---|
| | | | | Col T | BPR |
| 1 | Chiralpak® AD-3 column (3.0 µm, 50 × 4.6 mm) | A: $CO_2$<br>B: EtOH +0.05 % DEA | 5 % B to 40 % in 2 min and 40 % B hold 1.2 min, 5 % B hold 0.8 min | 4 | 4 |
| | | | | 35 | 100 |

Table: Analytical SFC data - $R_t$ means retention time (in minutes), $[M+H]^+$ means the protonated mass of the compound, method refers to the method used for (SFC)MS analysis of enantiomerically pure compounds. No. means number.

| Compound No. | SFC Method | Rt | $[M+H]^+$ |
|---|---|---|---|
| 5 | 1 | 1.98 | 641 |
| 6 | 1 | 1.87 | 641 |
| 7 | 1 | 2.26 | 640 |
| 8 | 1 | 2.11 | 640 |

NMR

$^1H$ NMR spectra were recorded on Bruker Avance III 400MHz and Avance NEO 400MHz spectrometers. $CDCl_3$ was used as solvent, unless otherwise mentioned. The chemical shifts are expressed in ppm relative to tetramethylsilane.

## Pharmacological Analysis

Biological Example 1

Terbium labeled Myeloid Cell Leukemia 1(Mcl-1) homogeneous time-resolved fluorescence (HTRF) binding assay utilizing the BIM BH3 peptide (H2N-(C/Cy5Mal) WIAQELRRIGDEFN-OH) as the binding partner for Mcl-1.

Apoptosis, or programmed cell death, ensures normal tissue homeostasis, and its dysregulation can lead to several human pathologies, including cancer. Whilst the extrinsic apoptosis pathway is initiated through the activation of cell-surface receptors, the intrinsic apoptosis pathway occurs at the mitochondrial outer membrane and is governed by the binding interactions between pro- and anti-apoptotic Bcl-2 family proteins, including Mcl-1. In many cancers, the anti-apoptotic Bcl-2 protein(s), such as the Mcl-1, are upregulated, and in this way the cancer cells can evade apoptosis. Thus, inhibition of the Bcl-2 protein(s), such as Mcl-1, may lead to apoptosis in cancer cells, providing a method for the treatment of said cancers.

This assay evaluated inhibition of the BH3 domain : Mcl-1 interaction by measuring the displacement of Cy5-labeled BIM BH3 peptide ($H_2N$-(C/Cy5Mal) WIAQELRRIGDEFN-OH) in the HTRF assay format.

Assay Procedure

The following assay and stock buffers were prepared for use in the assay: (a) Stock buffer: 10mM Tris-HCl, pH=7.5 + 150mM NaCl, filtered, sterilized, and stored at 4°C; and (b) 1X assay buffer, where the following ingredients were added fresh to stock buffer: 2 mM dithiothreitol (DTT), 0.0025% Tween-20, 0.1 mg/mL bovine serum albumin (BSA). The 1X Tb-Mcl-1 + Cy5 Bim peptide solution was prepared by diluting the protein stock solution using the 1X assay buffer (b) to 25 pM Tb-Mcl-1 and 8 nM Cy5 Bim peptide.

Using the Acoustic ECHO, 100 nL of 100x test compound(s) were dispensed into individual wells of a white 384-well Perkin Elmer Proxiplate, for a final compound concentration of 1x and final DMSO concentration of 1%. Inhibitor control and neutral control (NC, 100 nL of 100% DMSO) were stamped into columns 23 and 24 of assay plate, respectively. Into each well of the plate was then dispensed 10μL of the 1X Tb-Mcl-1 + Cy5 Bim peptide solution. The plate was centrifuged with a cover plate at 1000 rpm for 1 minute, then incubated for 60 minutes at room temperature with plates covered. The TR-FRET signal was read on an BMG PHERAStar FSX MicroPlate Reader at room temperature using the HTRF optic module (HTRF: excitation: 337nm, light source: laser, emission A: 665nm, emission B: 620nm, integration start: 60 μs, integration time: 400 μs).

Data Analysis

The BMG PHERAStar FSX MicroPlate Reader was used to measure fluorescence intensity at two emission wavelengths - 665nm and 620nm - and report relative fluorescence units (RFU) for both emissions, as well as a ratio of the emissions (665nm/620nm)*10,000. The RFU values were normalized to percent inhibition as follows:

$$\%\ inhibition = (((NC - IC) - (compound - IC)) / (NC - IC)) * 100$$

where IC (inhibitor control, low signal) = mean signal of 1X Tb-MCl-1 + Cy5 Bim peptide+ inhibitor control or 100% inhibition of Mcl-1; NC (neutral control, high signal) = mean signal 1X Tb-MCl-1 + Cy5 Bim peptide with DMSO only or 0% inhibition An 11-point dose response curve was generated to determine $IC_{50}$ values (using GenData) based on the following equation:

$$Y=Bottom + (Top-Bottom)/(1+10^{((logIC_{50}-X)*HillSlope)})$$

where Y = % inhibition in the presence of X inhibitor concentration; Top = 100% inhibition derived from the IC (mean signal of Mcl-1 + inhibitor control); Bottom = 0% inhibition derived from the NC (mean signal of Mcl-1 + DMSO); Hillslope = Hill coefficient; and $IC_{50}$ = concentration of compound with 50% inhibition in relation to top/neutral control (NC).

$$Ki = IC_{50} / (1 + [L]/Kd)$$

In this assay [L] = 8 nM and Kd = 10 nM

Representative compounds of the present invention were tested according to the procedure as described above, with results as listed in the Table below (n.d. means not determined).

| Compound | Tb-MCL1 $K_i$ (nM) |
|---|---|
| 1 | 0.04 |
| 2 | 3.28 |
| 3 | 0.10 |
| 4 | 1.57 |
| 5 | 5.21 |
| 6 | 445.48 |
| 7 | 0.95 |

(continued)

| Compound | Tb-MCL1 $K_i$ (nM) |
|---|---|
| 8 | 421.72 |

Biological Example 2

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

The Caspase-Glo® 3/7 Assay is a luminescent assay that measures caspase-3 and -7 activities in purified enzyme preparations or cultures of adherent or suspension cells. The assay provides a proluminescent caspase-3/7 substrate, which contains the tetrapeptide sequence DEVD. This substrate is cleaved to release aminoluciferin, a substrate of luciferase used in the production of light. Addition of the single Caspase-Glo® 3/7 Reagent in an "add-mix-measure" format results in cell lysis, followed by caspase cleavage of the substrate and generation of a "glow-type" luminescent signal.

This assay uses the MOLP-8 human multiple myeloma cell line, which is sensitive to MCL-1 inhibition.

Materials:

- Perkin Elmer Envision
- Multidrop 384 and small volume dispensing cassettes
- Centrifuge
- Countess automated cell counter
- Countess counting chamber slides
- Assay plate: ProxiPlate-384 Plus, White 384-shallow well Microplate
- Sealing tape: Topseal A plus
- T175 culture flask

| **Product** | **Units** | **Storage** |
|---|---|---|
| RPMI1640 (no L-Glutamine, no phenol red) | 500 mL | 4 °C |
| Foetal Bovine Serum (FBS) (Heat inactivated) | 500 mL | 4 °C |
| L-Glutamine (200 mM) | 100 ml | -20 °C |
| Gentamicin (50 mg/mL) | 100 mL | 4 °C |
| Caspase 3/7 Detection kit | 100 mL<br>10 × 10 mL | -20 °C |

Cell culture media:

| **MOLP8** | |
|---|---|
| | |
| RPMI-1640 medium | 500 mL |
| 20 % FBS (heat inactivated) | 120 mL |
| 2 mM L-Glutamine | 6.2 mL |
| 50 μg/mL Gentamicin | 620 μL |

(continued)

| MOLP8 | |
|---|---|
| | |
| Assay media | |
| | |
| RPMI-1640 medium | 500 mL |
| 10 % FBS (Heat inactivated) | 57 mL |
| 2 mM L-Glutamine | 5.7 mL |
| 50 μg/mL Gentamicin | 570 μL |

Cell culture:

Cell cultures were maintained between 0.2 and 2.0 $\times 10^6$ cells/mL. The cells were harvested by collection in 50 mL conical tubes. The cells were then pelleted at 500 g for 5 mins before removing supernatant and resuspension in fresh pre-warmed culture medium. The cells were counted and diluted as needed.

Caspase-Glo reagent:

The assay reagent was prepared by transferring the buffer solution to the substrate vial and mixing. The solution may be stored for up to 1 week at 4 °C with negligible loss of signal.

Assay procedure:

Compounds were delivered in assay-ready plates (Proxiplate) and stored at -20°C. Assays always include 1 reference compound plate containing reference compounds. The plates were spotted with 40 nL of compounds (0.5 % DMSO final in cells; serial dilution; 30 μM highest conc. 1/3 dilution, 10 doses, duplicates). The compounds were used at room temperature and 4 μL of pre-warmed media was added to all wells except column 2 and 23. The negative control was prepared by adding 1 % DMSO in media. The positive control was prepared by adding the appropriate positive control compound in final concentration of 60 μM in media. The plate was prepared by adding 4 μL negative control to column 23, 4 μL positive control to column 2 and 4 μL cell suspension to all wells in the plate. The plate with cells was then incubated at 37 °C for 2 hours. The assay signal reagent is the Caspase-Glo solution described above, and 8 μL was added to all wells. The plates were then sealed and measured after 30 minutes.

The activity of a test compound was calculated as percent change in apoptosis induction as follows:

LC

= median of the Low Control values

= Central Reference in Screener

= DMSO

= 0%

HC

= Median of the High Control values
= Scale Reference in Screener
= 30 μM of positive control
= 100 % apoptosis induction

$$\%\text{Effect } (AC_{50}) = 100 - ((\text{sample-LC}) / (\text{HC-LC})) * 100$$

$$\%Control = (sample / HC)*100$$

$$\%Control\ min = ((sample-LC) / (HC-LC)) *100$$

Table: Measured $AC_{50}$ for Representative Compounds of Formula (I). Averaged values are reported over all runs on all batches of a particular compound.

| Compound | MOLP8 Caspase-Glo $AC_{50}$ (μM) |
|---|---|
| 1 | 0.026 |
| 2 | 1.928 |
| 3 | 0.131 |
| 4 | 4.013 |
| 5 | 8.179 |
| 6 | >30 |
| 7 | 3.175 |
| 8 | 27.517 |

## Claims

**1.** A compound of Formula (I)

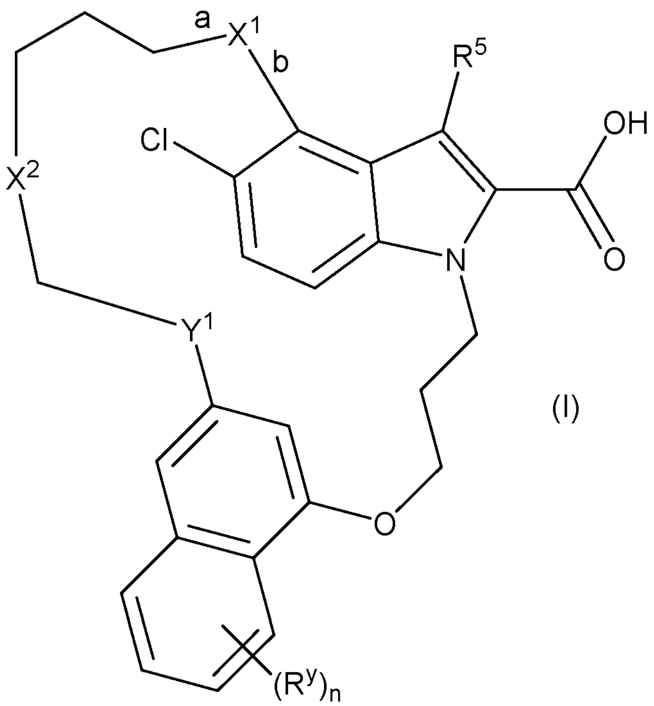

(I)

,

or a tautomer or a stereoisomeric form thereof, wherein

$X^1$ represents

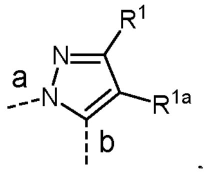

wherein 'a' and 'b' indicate how variable $X^1$ is attached to the remainder of the molecule;
$R^y$ represents halo;
n represents 0, 1 or 2;
$X^2$ represents

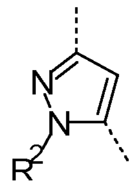

which can be attached to the remainder of the molecule in both directions;
or $X^2$ represents

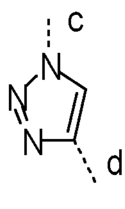

or

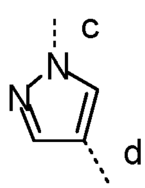

wherein 'c' and 'd' indicate how variable $X^2$ is attached to the remainder of the molecule;
$R^1$ represents hydrogen; or $C_{1-6}$alkyl optionally substituted with one substituent selected from the group consisting of $Het^1$, $-OR^3$, and $-NR^{4a}R^{4b}$;
$R^2$ represents hydrogen; methyl; or $C_{2-6}$alkyl optionally substituted with one substituent selected from the group consisting of $Het^1$, $-OR^3$, and $-NR^{4a}R^{4b}$;
$R^{1a}$ represents methyl or ethyl;
$R^3$ represents hydrogen, $C_{1-4}$alkyl, or $-C_{2-4}$alkyl-O-$C_{1-4}$alkyl;
$R^{4a}$ and $R^{4b}$ are each independently selected from the group consisting of hydrogen and $C_{1-4}$alkyl;
$R^5$ represents hydrogen; methyl; or $C_{2-6}$alkyl optionally substituted with one substituent selected from the group consisting of $C_{3-6}$cycloalkyl, $Het^1$, $-NR^{4a}R^{4b}$, and $-OR^3$;
$Het^1$ represents a 4- to 7-membered monocyclic fully saturated heterocyclyl containing one or two heteroatoms each independently selected from O, S, and N, wherein said S-atom might be substituted to form S(=O) or $S(=O)_2$; wherein said heterocyclyl is optionally substituted with one or two substituents each independently selected from the group consisting of halo, cyano, and -O-$C_{1-4}$alkyl;
$Y^1$ represents $-(CH_2)_m-$ or -S-;
m represents 1 or 2;
or a pharmaceutically acceptable salt, or a solvate thereof.

**2.** The compound according to claim 1, wherein
$X^2$ represents

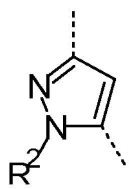

which can be attached to the remainder of the molecule in both directions.

**3.** The compound according to claim 1 or 2, wherein

n represents 0 or 1;
$R^1$ represents methyl;
$R^2$ represents methyl;
$R^{1a}$ represents methyl;
$R^5$ represents methyl;
$Y^1$ represents $-(CH_2)_m-$ or -S-;
m represents 1.

**4.** The compound according to claim 3, wherein
$Y^1$ represents $-(CH_2)_m-$.

**5.** The compound according to claim 4, wherein n is 0.

**6.** The compound according to claim 4, wherein n is 1.

**7.** The compound according to claim 1 or 2, wherein $Y^1$ represents $-(CH_2)_m-$, and m represents 1.

**8.** The compound according to any one of claims 1 to 7, wherein
$R^5$ represents methyl.

**9.** The compound according to claim 6, wherein $R^y$ represents fluoro.

**10.** A pharmaceutical composition comprising a compound as claimed in any one of claims 1 to 9 and a pharmaceutically acceptable carrier or diluent.

**11.** A process for preparing a pharmaceutical composition as defined in claim 10 comprising mixing a pharmaceutically acceptable carrier with a therapeutically effective amount of a compound according to any one of claims 1 to 9.

**12.** A compound as claimed in any one of claims 1 to 9 or a pharmaceutical composition as claimed in claim 10 for use as a medicament.

**13.** A compound as claimed in any one of claims 1 to 9 or a pharmaceutical composition as claimed in claim 10 for use in the prevention or treatment of cancer.

**14.** The compound or a pharmaceutical composition for use according to claim 13, wherein cancer is selected from prostate, lung, pancreatic, breast, ovarian, cervical, melanoma, B-cell chronic lymphocytic leukemia (CLL), acute myeloid leukemia (AML), and acute lymphoblastic leukemia (ALL).

**Patentansprüche**

**1.** Verbindung von Formel (I)

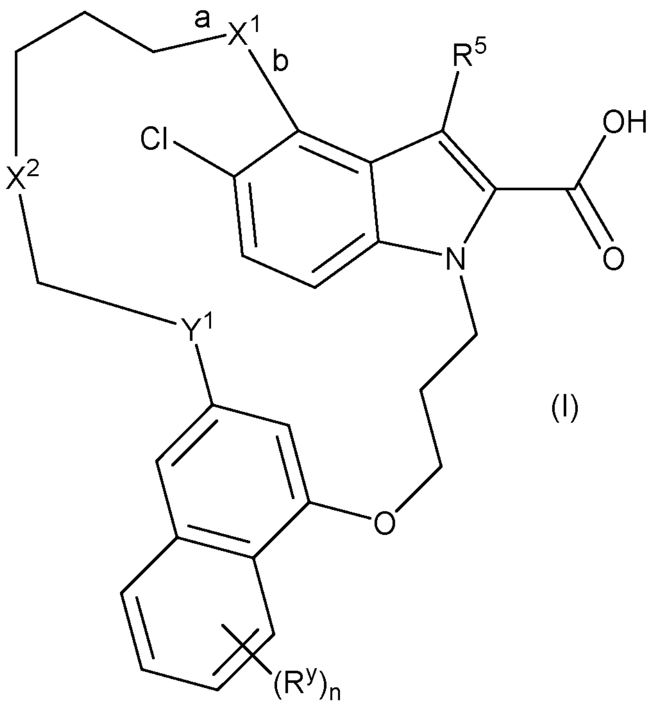

(I)

,

oder ein Tautomer oder eine stereoisomere Form davon, wobei

$X^1$ für

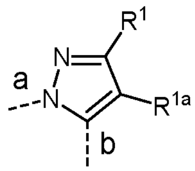

steht,
wobei "a" und "b" angeben, wie variabel $X^1$ an den Rest des Moleküls angelagert ist;
$R^y$ für Halogen steht;
n für 0, 1 oder 2 steht;
$X^2$ für

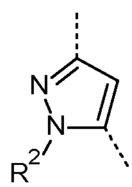

steht, das an den Rest des Moleküls in beiden Richtungen angelagert werden kann;
oder $X^2$ für

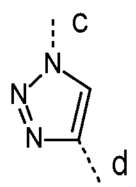

oder

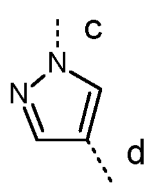

steht,
wobei "c" und "d" angeben, wie variabel $X^2$ an den Rest des Moleküls angelagert ist;
$R^1$ für Wasserstoff; oder $C_{1-6}$-Alkyl steht, optional mit einem Substituenten substituiert, der aus der Gruppe ausgewählt ist, bestehend aus $Het^1$, $-OR^3$, und $-NR^{4a}R^{4b}$;
$R^2$ für Wasserstoff; Methyl; oder $C_{2-6}$-Alkyl steht, optional mit einem Substituenten substituiert, der aus der Gruppe ausgewählt ist, bestehend aus $Het^1$, $-OR^3$ und $-NR^{4a}R^{4b}$;
$R^{1a}$ für Methyl oder Ethyl steht;
$R^3$ für Wasserstoff, $C_{1-4}$-Alkyl oder $-C_{2-4}$-Alkyl-O-$C_{1-4}$-Alkyl steht;
$R^{4a}$ und $R^{4b}$ jeweils unabhängig aus der Gruppe ausgewählt sind, bestehend aus Wasserstoff und $C_{1-4}$-Alkyl;
$R^5$ für Wasserstoff; Methyl; oder $C_{2-6}$-Alkyl steht, optional mit einem Substituenten substituiert, der aus der Gruppe ausgewählt ist, bestehend aus $C_{3-6}$-Cycloalkyl, $Het^1$, $-NR^{4a}R^{4b}$ und $-OR^3$;
$Het^1$ für ein 4- bis 7-gliedriges monocyclisches vollständig gesättigtes Heterocyclyl steht, das ein oder zwei Heteroatome enthält, die jeweils unabhängig aus O, S und N ausgewählt sind; wobei das S-Atom substituiert sein kann, um S(=O) oder $S(=O)_2$ auszubilden; wobei das Heterocyclyl optional mit einem oder zwei Substituenten substituiert ist, die jeweils unabhängig aus der Gruppe ausgewählt sind, bestehend aus Halogen, Cyano und $-O-C_{1-4}$-Alkyl;
$Y^1$ für $-(CH_2)_m-$ oder -S- steht;
m für 1 oder 2 steht;
oder ein pharmazeutisch unbedenkliches Salz oder ein Solvat davon.

**2.** Verbindung nach Anspruch 1, wobei

$X^2$ für

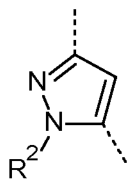

steht, das an den Rest des Moleküls in beiden Richtungen angelagert werden kann.

**3.** Verbindung nach Anspruch 1 oder 2, wobei

n für 0 oder 1 steht;
$R^1$ für Methyl steht;
$R^2$ für Methyl steht;
$R^{1a}$ für Methyl steht;
$R^5$ für Methyl steht;
$Y^1$ für $-(CH_2)_m-$ oder -S- steht;
m für 1 steht.

**4.** Verbindung nach Anspruch 3, wobei
$Y^1$ für $-(CH_2)_m-$ steht.

**5.** Verbindung nach Anspruch 4, wobei n 0 ist.

**6.** Verbindung nach Anspruch 4, wobei n 1 ist.

**7.** Verbindung nach Anspruch 1 oder 2, wobei $Y^1$ für $-(CH_2)_m-$ steht und m für 1 steht.

**8.** Verbindung nach einem der Ansprüche 1 bis 7, wobei
$R^5$ für Methyl steht.

**9.** Verbindung nach Anspruch 6, wobei $R^y$ für Fluor steht.

**10.** Pharmazeutische Zusammensetzung, umfassend eine Verbindung nach einem der Ansprüche 1 bis 9 und eine

pharmazeutisch unbedenkliche Trägersubstanz oder Verdünnungsmittel.

**11.** Verfahren zum Herstellen einer pharmazeutischen Zusammensetzung wie in Anspruch 10 definiert, umfassend ein Mischen eines pharmazeutisch unbedenklichen Trägers mit einer therapeutisch wirksamen Menge einer Verbindung nach einem der Ansprüche 1 bis 9.

**12.** Verbindung nach einem der Ansprüche 1 bis 9 oder eine pharmazeutische Zusammensetzung nach Anspruch 10 zur Verwendung als ein Medikament.

**13.** Verbindung nach einem der Ansprüche 1 bis 9 oder eine pharmazeutische Zusammensetzung nach Anspruch 10 zur Verwendung bei einer Vorbeugung oder Behandlung von Krebs.

**14.** Verbindung oder pharmazeutische Zusammensetzung zur Verwendung nach Anspruch 13, wobei der Krebs aus Prostata, Lunge, Bauchspeicheldrüse, Brust, Eierstock, Gebärmutterhals, Melanom, chronischer lymphatischer Leukämie (CLL) vom B-Zell-Typ, akuter myeloischer Leukämie (AML) und akuter lymphoblastischer Leukämie (ALL) ausgewählt ist.

**Revendications**

**1.** Composé de Formule (I)

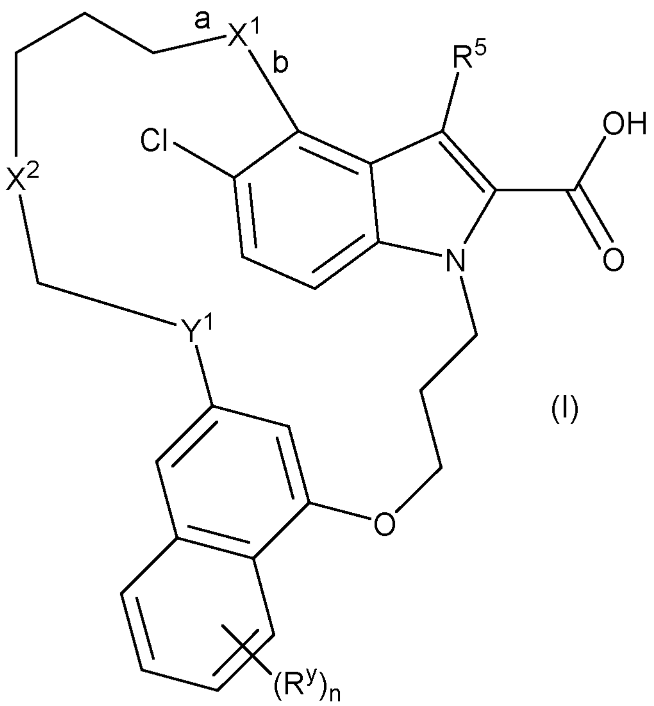

(I)

,

ou tautomère ou forme stéréoisomère de celui-ci, dans lequel

$X^1$ représente

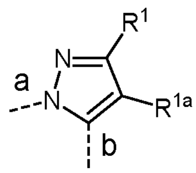

,

dans lequel ‘a’ et ’b’ indiquent comment la variable $X^1$ est liée au reste de la molécule ;

$R^y$ représente halo ;
n représente 0, 1 ou 2 ;
$X^2$ représente

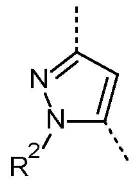

qui peut être lié au reste de la molécule dans les deux sens ;
ou $X^2$ représente

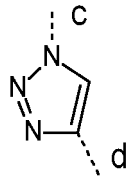
ou
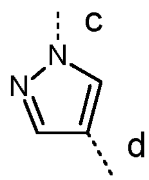

dans lequel 'c' et 'd' indiquent comment la variable $X^2$ est liée au reste de la molécule ;
$R^1$ représente hydrogène ; ou $C_{1-6}$alkyle facultativement substitué par un substituant choisi dans le groupe constitué de $Het^1$, $-OR^3$, et $-NR^{4a}R^{4b}$ ;
$R^2$ représente hydrogène ; méthyle ; ou $C_{2-6}$alkyle facultativement substitué par un substituant choisi dans le groupe constitué de $Het^1$, $-OR^3$, et - $NR^{4a}R^{4b}$ ;
$R^{1a}$ représente méthyle ou éthyle ;
$R^3$ représente hydrogène, $C_{1-4}$alkyle, ou $-C_{2-4}$alkyl-O-$C_{1-4}$alkyle ;
$R^{4a}$ et $R^{4b}$ sont chacun choisis indépendamment dans le groupe constitué d'hydrogène et $C_{1-4}$alkyle ;
$R^5$ représente hydrogène ; méthyle ; ou $C_{2-6}$alkyle facultativement substitué par un substituant choisi dans le groupe constitué de $C_{3-6}$cycloalkyle, $Het^1$, $-NR^{4a}R^{4b}$, et
$-OR^3$ ;
$Het^1$ représente un hétérocyclyle totalement saturé monocyclique à 4 à 7 chaînons contenant un ou deux hétéroatomes chacun choisis indépendamment parmi O, S, et N, dans lequel ledit atome S pourrait être substitué pour former S(=O) ou $S(=O)_2$ ; dans lequel ledit hétérocyclyle est facultativement substitué par un ou deux substituants chacun choisis indépendamment dans le groupe constitué de halo, cyano, et -O-$C_{1-4}$alkyle ;
$Y^1$ représente $-(CH_2)_m-$ ou -S- ;
m représente 1 ou 2 ;
ou un sel pharmaceutiquement acceptable, ou un solvate de celui-ci.

**2.** Composé selon la revendication 1, dans lequel

$X^2$ représente

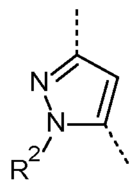

qui peut être lié au reste de la molécule dans les deux sens.

**3.** Composé selon la revendication 1 ou 2, dans lequel

n représente 0 ou 1 ;
$R^1$ représente méthyle ;

$R^2$ représente méthyle ;
$R^{1a}$ représente méthyle ;
$R^5$ représente méthyle ;
$Y^1$ représente $-(CH_2)_m-$ ou -S- ;
m représente 1.

**4.** Composé selon la revendication 3, dans lequel $Y^1$ représente $-(CH_2)_m-$.

**5.** Composé selon la revendication 4, dans lequel n vaut 0.

**6.** Composé selon la revendication 4, dans lequel n vaut 1.

**7.** Composé selon la revendication 1 ou 2, dans lequel $Y^1$ représente $-(CH_2)_m-$, et m représente 1.

**8.** Composé selon l'une quelconque des revendications 1 à 7, dans lequel $R^5$ représente méthyle.

**9.** Composé selon la revendication 6, dans lequel $R^y$ représente fluoro.

**10.** Composition pharmaceutique comprenant un composé selon l'une quelconque des revendications 1 à 9 et un support ou diluant pharmaceutiquement acceptable.

**11.** Procédé permettant de préparer une composition pharmaceutique telle que définie dans la revendication 10 comprenant le mélange d'un support pharmaceutiquement acceptable avec une quantité thérapeutiquement efficace d'un composé selon l'une quelconque des revendications 1 à 9.

**12.** Composé selon l'une quelconque des revendications 1 à 9 ou composition pharmaceutique selon la revendication 10 pour une utilisation en tant que médicament.

**13.** Composé selon l'une quelconque des revendications 1 à 9 ou composition pharmaceutique selon la revendication 10 pour une utilisation dans la prévention ou le traitement d'un cancer.

**14.** Composé ou composition pharmaceutique pour une utilisation selon la revendication 13, dans lequel le cancer est choisi parmi cancer de la prostate, du poumon, du pancréas, du sein, de l'ovaire, du col de l'utérus, mélanome, leucémie lymphoïde chronique à lymphocytes B (LLC), leucémie myéloïde aiguë (LMA), et leucémie lymphoblastique aiguë (LLA).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018178226 A
- WO 2017182625 A
- WO 2018178227 A
- WO 2020063792 A
- WO 2020103864 A


### Non-patent literature cited in the description


- **CHENG et al.** *eLife,* 2016, vol. 5, e17755
- **JULIAN et al.** *Cell Death and Differentiation,* 2017, vol. 24, 1380-1389
- **HANAHAN ; WEINBERG.** *Cell,* 2011, 1-44
- **BEROUKHIM et al.** *Nature,* 2010, vol. 463 (7283), 899-905
- **YECIES et al.** *Blood,* 2010, vol. 115 (16), 3304-3313
- **ROBERTS et al.** *NEJM,* 2016, vol. 374, 311-322
- **KOTSCHY et al.** *Nature,* 2016, vol. 538, 477-486
- **MERINO et al.** *Sci. Transl. Med,* 2017, vol. 9
- **CHEN et al.** *JCI,* 2018, vol. 128 (1), 500-516
- **WANG et al.** *Genes and Dev,* 2013, vol. 27, 1351-1364
- **STEIMER et al.** *Blood,* 2009, vol. 113, 2805-2815
- **ELIEL, E.L. ; WILEN, S. H.** Stereochemistry of Organic Compounds. John Wiley and Sons, Inc, 1994
- **CHARRON, CARLIE L. et al.** *Tetrahedron Lett.,* 2016, vol. 57 (37), 4119-4127
- **AUSTIN R. et al.** *Cancer Letters,* 2016
- *CHEMICAL ABSTRACTS,* 1784533-05-6
- *CHEMICAL ABSTRACTS,* 53199-31-8
- *CHEMICAL ABSTRACTS,* 887919-35-9
- *CHEMICAL ABSTRACTS,* 61676-62-8
- *CHEMICAL ABSTRACTS,* 2820-37-3
- *CHEMICAL ABSTRACTS,* 89031-84-5
- *CHEMICAL ABSTRACTS,* 2143010-96-0
- *CHEMICAL ABSTRACTS,* 2152839-94-4
- *CHEMICAL ABSTRACTS,* 2092726-85-5
- *CHEMICAL ABSTRACTS,* 2109428-60-4
- **T. W. GREENE ; P. G. M. WUTS.** Protective Groups in Organic Synthesis. Wiley, 2007
- **GENNARO et al.** Remington's Pharmaceutical Sciences. Mack Publishing Company, 1990
- *CHEMICAL ABSTRACTS,* 1172589-41-1
- *CHEMICAL ABSTRACTS,* 3952-66-7
- *CHEMICAL ABSTRACTS,* 26386-88-9
- *CHEMICAL ABSTRACTS,* 33821-94-2
- *CHEMICAL ABSTRACTS,* 10465-81-3